# EUROPEAN PATENT APPLICATION

(11) **EP 3 670 498 A1**
(43) Date of publication of application: **24.06.2020**
(21) Application number: 18212936.1
(22) Date of filing: 17.12.2018
(51) Int. Cl.: C07D 229/02, C07D 249/06, C07D 295/096, C07D 295/135, A61P 31/14

(54) **COMPOUNDS FOR TREATMENT OF HEPACI VIRUS INFECTION AND METHOD FOR DETERMINING THERAPY OF HEPACI VIRUS INFECTION, IN PARTICULAR, HCV INFECTION**

(71) Applicant: Gottfried Wilhelm Leibniz Universität Hannover, 30167 Hannover (DE); Twincore Zentrum für Experimentelle und Klinische Infektionsforschung GmbH, 30625 Hannover (DE)
(72) Inventor: PIETSCHMANN, Thomas, 30559 Hannover (DE); BANDA, Dominic, 9000 Ghent (BE); KIRSCHNING, Andreas, 29221 Celle (DE); SOLODENKO, Wladimir, 30177 Hannover (DE)
(74) Representative: Gramm, Lins & Partner Patent- und Rechtsanwälte PartGmbB

(57) **Abstract**

In a first aspect, the present invention relates to new compounds based on diphenylpiperazine and diphenylpiperidine structures. In particular, the present invention provides new flunarizine derivatives having improved hepaci virus infection inhibitory activity. In a further aspect, the present invention relates to a pharmaceutical composition containing said compound as well as the use of said pharmaceutical composition and the compounds according to the present invention in preventing or treating hepaci virus infection, in particular, HCV virus infection, like HCV of genotype 2. Moreover, a method for determining effectiveness of prophylactic or therapeutic treatment of hepaci virus, like HCV infection as well as a method for determining the therapy regimen of an individual afflicted with hepaci virus infection including HCV infection is provided. Said method is based on determining the sequence or interfacial hydrophobicity of the hepaci virus E1 protein. This may include determining the presence of mutations at predetermined positions of the E1 sequence. Based on determining the interfacial hydrophobicity of the E1 protein, the sensitivity to a diphenylpiperazine or diphenylpiperidine based hepaci virus inhibitor as well as a phenothiazine and cycloheptenepiperidine based hepaci virus inhibitor can be determined. When the central hydrophobicity region is disrupted or the hydrophobicity is below zero applying the Wimley-White hydropathy plot, or the mutations at positions 290, 299, 301 and 310 of SEQ ID No. 1 are present, it is submitted that the sensitivity against said compounds is reduced. Hence, it is possible to determine the therapy regimen of an individual afflicted with hepaci virus infection or being a risk of being afflicted with hepaci virus infection, in particular HCV infection like HCV genotype 2 infection.

SEQUENCE LISTING

<110>  Gottfried Wilhelm Leibniz Universitaet Hannover
       Twincore Zentrum fuer Experimentelle und Klinische
       Infektionsforschung GmbH

<120>  Compounds for treatment of heapci virus infection and method for
       determining therapy of hepaci virus infection, in particular, HCV
        infection

<130>  2674-216 EP-1

<160>  1

<170>  PatentIn version 3.5

<210>  1
<211>  3033
<212>  PRT
<213>  hepatitis C virus

<400>  1

Met Ser Thr Asn Pro Lys Pro Gln Arg Lys Thr Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Gly Arg Arg Gln Pro
    50                  55                  60

Ile Pro Lys Asp Arg Arg Ser Thr Gly Lys Ser Trp Gly Lys Pro Gly
65                  70                  75                  80

Tyr Pro Trp Pro Leu Tyr Gly Asn Glu Gly Leu Gly Trp Ala Gly Trp
                85                  90                  95

Leu Leu Ser Pro Arg Gly Ser Arg Pro Ser Trp Gly Pro Asn Asp Pro
            100                 105                 110

Arg His Arg Ser Arg Asn Val Gly Lys Val Ile Asp Thr Leu Thr Cys
        115                 120                 125

Gly Phe Ala Asp Leu Met Gly Tyr Ile Pro Val Val Gly Ala Pro Leu
    130                 135                 140

Gly Gly Val Ala Arg Ala Leu Ala His Gly Val Arg Val Leu Glu Asp
145                 150                 155                 160

Gly Val Asn Phe Ala Thr Gly Asn Leu Pro Gly Cys Ser Phe Ser Ile
                165                 170                 175

Phe Leu Leu Ala Leu Leu Ser Cys Ile Thr Thr Pro Val Ser Ala Ala
            180                 185                 190

Glu Val Lys Asn Ile Ser Thr Gly Tyr Met Val Thr Asn Asp Cys Thr
        195                 200                 205

Asn Asp Ser Ile Thr Trp Gln Leu Gln Ala Ala Val Leu His Val Pro
    210                 215                 220

Gly Cys Val Pro Cys Glu Lys Val Gly Asn Ala Ser Gln Cys Trp Ile
225                 230                 235                 240

Pro Val Ser Pro Asn Val Ala Val Gln Arg Pro Gly Ala Leu Thr Gln
                245                 250                 255

Gly Leu Arg Thr His Ile Asp Met Val Val Met Ser Ala Thr Leu Cys
            260                 265                 270

Ser Ala Leu Tyr Val Gly Asp Leu Cys Gly Gly Val Met Leu Ala Ala
        275                 280                 285

Gln Met Phe Ile Val Ser Pro Gln His His Trp Phe Val Gln Asp Cys
    290                 295                 300

Asn Cys Ser Ile Tyr Pro Gly Thr Ile Thr Gly His Arg Met Ala Trp
305                 310                 315                 320

Asp Met Met Met Asn Trp Ser Pro Thr Ala Thr Met Ile Leu Ala Tyr
                325                 330                 335

Ala Met Arg Val Pro Glu Val Ile Ile Asp Ile Ile Ser Gly Ala His
            340                 345                 350

Trp Gly Val Met Phe Gly Leu Ala Tyr Phe Ser Met Gln Gly Ala Trp
        355                 360                 365

Ala Lys Val Val Val Ile Leu Leu Leu Ala Ala Gly Val Asp Ala Arg
    370                 375                 380

Thr His Thr Val Gly Gly Ser Ala Ala Gln Thr Thr Gly Arg Leu Thr
385                 390                 395                 400

Ser Leu Phe Asp Met Gly Pro Arg Gln Lys Ile Gln Leu Val Asn Thr
                405                 410                 415

Asn Gly Ser Trp His Ile Asn Arg Thr Ala Leu Asn Cys Asn Asp Ser
        420                 425                 430

Leu His Thr Gly Phe Ile Ala Ser Leu Phe Tyr Thr His Ser Phe Asn
    435                 440                 445

Ser Ser Gly Cys Pro Glu Arg Met Ser Ala Cys Arg Ser Ile Glu Ala
    450                 455                 460

Phe Arg Val Gly Trp Gly Ala Leu Gln Tyr Glu Asp Asn Val Thr Asn
465                 470                 475                 480

Pro Glu Asp Met Arg Pro Tyr Cys Trp His Tyr Pro Pro Arg Gln Cys
                485                 490                 495

Gly Val Val Ser Ala Lys Thr Val Cys Gly Pro Val Tyr Cys Phe Thr
            500                 505                 510

Pro Ser Pro Val Val Val Gly Thr Thr Asp Arg Leu Gly Ala Pro Thr
        515                 520                 525

Tyr Thr Trp Gly Glu Asn Glu Thr Asp Val Phe Leu Leu Asn Ser Thr
    530                 535                 540

Arg Pro Pro Leu Gly Ser Trp Phe Gly Cys Thr Trp Met Asn Ser Ser
545                 550                 555                 560

Gly Tyr Thr Lys Thr Cys Gly Ala Pro Pro Cys Arg Thr Arg Ala Asp
                565                 570                 575

Phe Asn Ala Ser Thr Asp Leu Leu Cys Pro Thr Asp Cys Phe Arg Lys
            580                 585                 590

His Pro Asp Thr Thr Tyr Leu Lys Cys Gly Ser Gly Pro Trp Leu Thr
        595                 600                 605

Pro Arg Cys Leu Ile Asp Tyr Pro Tyr Arg Leu Trp His Tyr Pro Cys
    610                 615                 620

Thr Val Asn Tyr Thr Ile Phe Lys Ile Arg Met Tyr Val Gly Gly Val
625                 630                 635                 640

Glu His Arg Leu Thr Ala Ala Cys Asn Phe Thr Arg Gly Asp Arg Cys
                645                 650                 655

Asn Leu Glu Asp Arg Asp Arg Ser Gln Leu Ser Pro Leu Leu His Ser
            660                 665                 670

Thr Thr Glu Trp Ala Ile Leu Pro Cys Ser Tyr Ser Asp Leu Pro Ala
        675                 680                 685

Leu Ser Thr Gly Leu Leu His Leu His Gln Asn Ile Val Asp Val Gln
    690                 695                 700

Phe Met Tyr Gly Leu Ser Pro Ala Leu Thr Lys Tyr Ile Val Arg Trp
705                 710                 715                 720

Glu Trp Val Ile Leu Leu Phe Leu Leu Leu Ala Asp Ala Arg Val Cys
                725                 730                 735

Ala Cys Leu Trp Met Leu Ile Leu Leu Gly Gln Ala Glu Ala Ala Leu
            740                 745                 750

Glu Lys Leu Val Ile Leu His Ala Ala Ser Ala Ala Ser Cys Asn Gly
        755                 760                 765

Phe Leu Tyr Phe Val Ile Phe Phe Val Ala Ala Trp Tyr Ile Lys Gly
    770                 775                 780

Arg Val Val Pro Leu Ala Thr Tyr Ser Leu Thr Gly Leu Trp Ser Phe
785                 790                 795                 800

Ser Leu Leu Leu Leu Ala Leu Pro Gln Gln Ala Tyr Ala Tyr Asp Ala
                805                 810                 815

Ser Val His Gly Gln Ile Gly Ala Ala Leu Leu Val Met Ile Thr Leu
            820                 825                 830

Phe Thr Leu Thr Pro Gly Tyr Lys Thr Leu Leu Ser Arg Phe Leu Trp
        835                 840                 845

Trp Leu Cys Tyr Leu Leu Thr Leu Gly Glu Ala Met Val Gln Glu Trp
    850                 855                 860

Ala Pro Pro Met Gln Val Arg Gly Gly Arg Asp Gly Ile Ile Trp Ala
865                 870                 875                 880

Val Ala Ile Phe Tyr Pro Gly Val Val Phe Asp Ile Thr Lys Trp Leu
                885                 890                 895

Leu Ala Val Leu Gly Pro Ala Tyr Leu Leu Lys Gly Ala Leu Thr Arg
            900                 905                 910

Val Pro Tyr Phe Val Arg Ala His Ala Leu Leu Arg Met Cys Thr Met

        915                 920                 925

Ala Arg His Leu Ala Gly Gly Arg Tyr Val Gln Met Ala Leu Leu Ala
    930                 935                 940

Leu Gly Arg Trp Thr Gly Thr Tyr Ile Tyr Asp His Leu Thr Pro Met
945                 950                 955                 960

Ser Asp Trp Ala Ala Ser Gly Leu Arg Asp Leu Ala Val Ala Val Glu
                965                 970                 975

Pro Ile Ile Phe Ser Pro Met Glu Lys Lys Val Ile Val Trp Gly Ala
            980                 985                 990

Glu Thr Ala Ala Cys Gly Asp Ile Leu His Gly Leu Pro Val Ser Ala
        995                 1000                1005

Arg Leu Gly Arg Glu Val Leu Leu Gly Pro Ala Asp Gly Tyr Thr
    1010                1015                1020

Ser Lys Gly Trp Ser Leu Leu Ala Pro Ile Thr Ala Tyr Ala Gln
    1025                1030                1035

Gln Thr Arg Gly Leu Leu Gly Thr Ile Val Val Ser Met Thr Gly
    1040                1045                1050

Arg Asp Lys Thr Glu Gln Ala Gly Glu Ile Gln Val Leu Ser Thr
    1055                1060                1065

Val Thr Gln Ser Phe Leu Gly Thr Ser Ile Ser Gly Val Leu Trp
    1070                1075                1080

Thr Val Tyr His Gly Ala Gly Asn Lys Thr Leu Ala Gly Ser Arg
    1085                1090                1095

Gly Pro Val Thr Gln Met Tyr Ser Ser Ala Glu Gly Asp Leu Val
    1100                1105                1110

Gly Trp Pro Ser Pro Pro Gly Thr Lys Ser Leu Glu Pro Cys Thr
    1115                1120                1125

Cys Gly Ala Val Asp Leu Tyr Leu Val Thr Arg Asn Ala Asp Val
    1130                1135                1140

Ile Pro Ala Arg Arg Arg Gly Asp Lys Arg Gly Ala Leu Leu Ser
    1145                1150                1155

Pro Arg Pro Leu Ser Thr Leu Lys Gly Ser Ser Gly Gly Pro Val
    1160                1165                1170

Leu Cys Pro Arg Gly His Ala Val Gly Val Phe Arg Ala Ala Val
    1175                1180                1185

Cys Ser Arg Gly Val Ala Lys Ser Ile Asp Phe Ile Pro Val Glu
    1190                1195                1200

Thr Leu Asp Ile Val Thr Arg Ser Pro Thr Phe Ser Asp Asn Ser
    1205                1210                1215

Thr Pro Pro Ala Val Pro Gln Thr Tyr Gln Val Gly Tyr Leu His
    1220                1225                1230

Ala Pro Thr Gly Ser Gly Lys Ser Thr Lys Val Pro Val Ala Tyr
    1235                1240                1245

Ala Ala Gln Gly Tyr Lys Val Leu Val Leu Asn Pro Ser Val Ala
    1250                1255                1260

Ala Thr Leu Gly Phe Gly Ala Tyr Leu Ser Lys Ala His Gly Ile
    1265                1270                1275

Asn Pro Asn Ile Arg Thr Gly Val Arg Thr Val Thr Thr Gly Ala
    1280                1285                1290

Pro Ile Thr Tyr Ser Thr Tyr Gly Lys Phe Leu Ala Asp Gly Gly
    1295                1300                1305

Cys Ala Gly Gly Ala Tyr Asp Ile Ile Ile Cys Asp Glu Cys His
    1310                1315                1320

Ala Val Asp Ser Thr Thr Ile Leu Gly Ile Gly Thr Val Leu Asp
    1325                1330                1335

Gln Ala Glu Thr Ala Gly Val Arg Leu Thr Val Leu Ala Thr Ala
    1340                1345                1350

Thr Pro Pro Gly Ser Val Thr Thr Pro His Pro Asn Ile Glu Glu
    1355                1360                1365

Val Ala Leu Gly Gln Glu Gly Glu Ile Pro Phe Tyr Gly Arg Ala
    1370                1375                1380

Ile Pro Leu Ser Tyr Ile Lys Gly Gly Arg His Leu Ile Phe Cys
    1385                1390                1395

His Ser Lys Lys Lys Cys Asp Glu Leu Ala Ala Ala Leu Arg Gly
    1400                1405                1410

Met Gly Leu Asn Ser Val Ala Tyr Tyr Arg Gly Leu Asp Val Ser
    1415                1420                1425

Val Ile Pro Thr Gln Gly Asp Val Val Val Val Ala Thr Asp Ala
    1430                1435                1440

Leu Met Thr Gly Tyr Thr Gly Asp Phe Asp Ser Val Ile Asp Cys
    1445                1450                1455

Asn Val Ala Val Thr Gln Val Val Asp Phe Ser Leu Asp Pro Thr
    1460                1465                1470

Phe Thr Ile Thr Thr Gln Ile Val Pro Gln Asp Ala Val Ser Arg
    1475                1480                1485

Ser Gln Arg Arg Gly Arg Thr Gly Arg Gly Arg Leu Gly Ile Tyr
    1490                1495                1500

Arg Tyr Val Ser Thr Gly Glu Arg Ala Ser Gly Met Phe Asp Ser
    1505                1510                1515

Val Val Leu Cys Glu Cys Tyr Asp Ala Gly Ala Ala Trp Tyr Glu
    1520                1525                1530

Leu Thr Pro Ser Glu Thr Thr Val Arg Leu Arg Ala Tyr Phe Asn
    1535                1540                1545

Thr Pro Gly Leu Pro Val Cys Gln Asp His Leu Glu Phe Trp Glu
    1550                1555                1560

Ala Val Phe Thr Gly Leu Thr His Ile Asp Ala His Phe Leu Ser
    1565                1570                1575

Gln Thr Lys Gln Ser Gly Glu Asn Phe Ala Tyr Leu Thr Ala Tyr
    1580                1585                1590

Gln Ala Thr Val Cys Ala Arg Ala Lys Ala Pro Pro Pro Ser Trp
    1595                1600                1605

Asp Val Met Trp Lys Cys Leu Thr Arg Leu Lys Pro Thr Leu Val
    1610                1615                1620

Gly Pro Thr Pro Leu Leu Tyr Arg Leu Gly Ser Val Thr Asn Glu
    1625                1630                1635

Val Thr Leu Thr His Pro Val Thr Lys Tyr Ile Ala Thr Cys Met
    1640                1645                1650

Gln Ala Asp Leu Glu Val Met Thr Ser Thr Trp Val Leu Ala Gly
    1655                1660                1665

Gly Val Leu Ala Ala Val Ala Ala Tyr Cys Leu Ala Thr Gly Cys
    1670                1675                1680

Val Cys Ile Ile Gly Arg Leu His Ile Asn Gln Arg Ala Val Val
    1685                1690                1695

Ala Pro Asp Lys Glu Val Leu Tyr Glu Ala Phe Asp Glu Met Glu
    1700                1705                1710

Glu Cys Ala Ser Arg Ala Ala Leu Ile Glu Glu Gly Gln Arg Ile
    1715                1720                1725

Ala Glu Met Leu Lys Ser Lys Ile Gln Gly Leu Leu Gln Gln Ala
    1730                1735                1740

Ser Lys Gln Ala Gln Asp Ile Gln Pro Thr Val Gln Ala Ser Trp
    1745                1750                1755

Pro Lys Val Glu Gln Phe Trp Ala Lys His Met Trp Asn Phe Ile
    1760                1765                1770

Ser Gly Ile Gln Tyr Leu Ala Gly Leu Ser Thr Leu Pro Gly Asn
    1775                1780                1785

Pro Ala Val Ala Ser Met Met Ala Phe Ser Ala Ala Leu Thr Ser
    1790                1795                1800

Pro Leu Ser Thr Ser Thr Thr Ile Leu Leu Asn Ile Leu Gly Gly
    1805                1810                1815

Trp Leu Ala Ser Gln Ile Ala Pro Pro Ala Gly Ala Thr Gly Phe
    1820                1825                1830

Val Val Ser Gly Leu Val Gly Ala Ala Val Gly Ser Ile Gly Leu
    1835                1840                1845

Gly Lys Val Leu Val Asp Ile Leu Ala Gly Tyr Gly Ala Gly Ile
    1850                1855                1860

Ser Gly Ala Leu Val Ala Phe Lys Ile Met Ser Gly Glu Lys Pro

1865                1870                1875

Ser Met  Glu Asp Val Val Asn  Leu Leu Pro Gly Ile  Leu Ser Pro
    1880                1885                1890

Gly Ala  Leu Val Val Gly Val  Ile Cys Ala Ala Ile  Leu Arg Arg
    1895                1900                1905

His Val  Gly Pro Gly Glu Gly  Ala Val Gln Trp Met  Asn Arg Leu
    1910                1915                1920

Ile Ala  Phe Ala Ser Arg Gly  Asn His Val Ala Pro  Thr His Tyr
    1925                1930                1935

Val Thr  Glu Ser Asp Ala Ser  Gln Arg Val Thr Gln  Leu Leu Gly
    1940                1945                1950

Ser Leu  Thr Ile Thr Ser Leu  Leu Arg Arg Leu His  Asn Trp Ile
    1955                1960                1965

Thr Glu  Asp Cys Pro Ile Pro  Cys Gly Gly Ser Trp  Leu Arg Asp
    1970                1975                1980

Val Trp  Asp Trp Val Cys Thr  Ile Leu Thr Asp Phe  Lys Asn Trp
    1985                1990                1995

Leu Thr  Ser Lys Leu Phe Pro  Lys Met Pro Gly Leu  Pro Phe Val
    2000                2005                2010

Ser Cys  Gln Lys Gly Tyr Lys  Gly Val Trp Ala Gly  Thr Gly Ile
    2015                2020                2025

Met Thr  Thr Arg Cys Pro Cys  Gly Ala Asn Ile Ser  Gly Asn Val
    2030                2035                2040

Arg Leu  Gly Ser Met Arg Ile  Thr Gly Pro Lys Thr  Cys Met Asn
    2045                2050                2055

Ile Trp  Gln Gly Thr Phe Pro  Ile Asn Cys Tyr Thr  Glu Gly Gln
    2060                2065                2070

Cys Val  Pro Lys Pro Ala Pro  Asn Phe Lys Val Ala  Ile Trp Arg
    2075                2080                2085

Val Ala  Ala Ser Glu Tyr Ala  Glu Val Thr Gln His  Gly Ser Tyr
    2090                2095                2100

His Tyr Ile Thr Gly Leu Thr Thr Asp Asn Leu Lys Val Pro Cys
    2105                2110                2115

Gln Leu Pro Ser Pro Glu Phe Phe Ser Trp Val Asp Gly Val Gln
    2120                2125                2130

Ile His Arg Phe Ala Pro Thr Pro Lys Pro Phe Phe Arg Asp Glu
    2135                2140                2145

Val Ser Phe Cys Val Gly Leu Asn Ser Phe Val Val Gly Ser Gln
    2150                2155                2160

Leu Pro Cys Asp Pro Glu Pro Asp Thr Asp Val Leu Met Ser Met
    2165                2170                2175

Leu Thr Asp Pro Ser His Ile Thr Ala Glu Thr Ala Ala Arg Arg
    2180                2185                2190

Leu Ala Arg Gly Ser Pro Pro Ser Glu Ala Ser Ser Ser Ala Ser
    2195                2200                2205

Gln Leu Ser Ala Pro Ser Leu Arg Ala Thr Cys Thr Thr His Gly
    2210                2215                2220

Lys Ala Tyr Asp Val Asp Met Val Asp Ala Asn Leu Phe Met Gly
    2225                2230                2235

Gly Asp Val Thr Arg Ile Glu Ser Gly Ser Lys Val Val Val Leu
    2240                2245                2250

Asp Ser Leu Asp Pro Met Val Glu Glu Arg Ser Asp Leu Glu Pro
    2255                2260                2265

Ser Ile Pro Ser Glu Tyr Met Leu Pro Lys Lys Arg Phe Pro Pro
    2270                2275                2280

Ala Leu Pro Ala Trp Ala Arg Pro Asp Tyr Asn Pro Pro Leu Val
    2285                2290                2295

Glu Ser Trp Lys Arg Pro Asp Tyr Gln Pro Ala Thr Val Ala Gly
    2300                2305                2310

Cys Ala Leu Pro Pro Pro Arg Lys Thr Pro Thr Pro Pro Pro Arg
    2315                2320                2325

Arg Arg Arg Thr Val Gly Leu Ser Glu Asp Ser Ile Gly Asp Ala
    2330                2335                2340

Leu Gln Gln Leu Ala Ile Lys Ser Phe Gly Gln Pro Pro Pro Ser
    2345                2350                2355

Gly Asp Ser Gly Leu Ser Thr Gly Ala Gly Ala Ala Asp Ser Gly
    2360                2365                2370

Ser Gln Thr Pro Pro Asp Glu Leu Ala Leu Ser Glu Thr Gly Ser
    2375                2380                2385

Ile Ser Ser Met Pro Pro Leu Glu Gly Glu Leu Gly Asp Pro Asp
    2390                2395                2400

Leu Glu Pro Glu Gln Val Glu Pro Gln Pro Pro Pro Gln Gly Gly
    2405                2410                2415

Val Ala Ala Pro Gly Ser Asp Ser Gly Ser Trp Ser Thr Cys Ser
    2420                2425                2430

Glu Glu Asp Asp Ser Val Val Cys Cys Ser Met Ser Tyr Ser Trp
    2435                2440                2445

Thr Gly Ala Leu Ile Thr Pro Cys Ser Pro Glu Glu Glu Lys Leu
    2450                2455                2460

Pro Ile Asn Pro Leu Ser Asn Ser Leu Leu Arg Tyr His Asn Lys
    2465                2470                2475

Val Tyr Cys Thr Thr Thr Lys Ser Ala Ser Leu Arg Ala Lys Lys
    2480                2485                2490

Val Thr Phe Asp Arg Met Gln Val Leu Asp Ser Tyr Tyr Asp Ser
    2495                2500                2505

Val Leu Lys Asp Ile Lys Leu Ala Ala Ser Lys Val Thr Ala Arg
    2510                2515                2520

Leu Leu Thr Met Glu Glu Ala Cys Gln Leu Thr Pro Pro His Ser
    2525                2530                2535

Ala Arg Ser Lys Tyr Gly Phe Gly Ala Lys Glu Val Arg Ser Leu
    2540                2545                2550

Ser Gly Arg Ala Val Asn His Ile Lys Ser Val Trp Lys Asp Leu
    2555                2560                2565

Leu Glu Asp Ser Glu Thr Pro Ile Pro Thr Thr Ile Met Ala Lys
    2570                2575                2580

Asn Glu Val Phe Cys Val Asp Pro Thr Lys Gly Gly Lys Lys Ala
    2585                2590                2595

Ala Arg Leu Ile Val Tyr Pro Asp Leu Gly Val Arg Val Cys Glu
    2600                2605                2610

Lys Met Ala Leu Tyr Asp Ile Thr Gln Lys Leu Pro Gln Ala Val
    2615                2620                2625

Met Gly Ala Ser Tyr Gly Phe Gln Tyr Ser Pro Ala Gln Arg Val
    2630                2635                2640

Glu Phe Leu Leu Lys Ala Trp Ala Glu Lys Lys Asp Pro Met Gly
    2645                2650                2655

Phe Ser Tyr Asp Thr Arg Cys Phe Asp Ser Thr Val Thr Glu Arg
    2660                2665                2670

Asp Ile Arg Thr Glu Glu Ser Ile Tyr Arg Ala Cys Ser Leu Pro
    2675                2680                2685

Glu Glu Ala His Thr Ala Ile His Ser Leu Thr Glu Arg Leu Tyr
    2690                2695                2700

Val Gly Gly Pro Met Phe Asn Ser Lys Gly Gln Thr Cys Gly Tyr
    2705                2710                2715

Arg Arg Cys Arg Ala Ser Gly Val Leu Thr Thr Ser Met Gly Asn
    2720                2725                2730

Thr Ile Thr Cys Tyr Val Lys Ala Leu Ala Ala Cys Lys Ala Ala
    2735                2740                2745

Gly Ile Ile Ala Pro Thr Met Leu Val Cys Gly Asp Asp Leu Val
    2750                2755                2760

Val Ile Ser Glu Ser Gln Gly Thr Glu Glu Asp Glu Arg Asn Leu
    2765                2770                2775

Arg Ala Phe Thr Glu Ala Met Thr Arg Tyr Ser Ala Pro Pro Gly
    2780                2785                2790

Asp Pro Pro Arg Pro Glu Tyr Asp Leu Glu Leu Ile Thr Ser Cys
    2795                2800                2805

Ser Ser Asn Val Ser Val Ala Leu Gly Pro Gln Gly Arg Arg Arg

2810                    2815                    2820

Tyr Tyr Leu Thr Arg Asp Pro Thr Thr Pro Ile Ala Arg Ala Ala
    2825                    2830                    2835

Trp Glu Thr Val Arg His Ser Pro Val Asn Ser Trp Leu Gly Asn
    2840                    2845                    2850

Ile Ile Gln Tyr Ala Pro Thr Ile Trp Ala Arg Met Val Leu Met
    2855                    2860                    2865

Thr His Phe Phe Ser Ile Leu Met Ala Gln Asp Thr Leu Asp Gln
    2870                    2875                    2880

Asn Leu Asn Phe Glu Met Tyr Gly Ala Val Tyr Ser Val Ser Pro
    2885                    2890                    2895

Leu Asp Leu Pro Ala Ile Ile Glu Arg Leu His Gly Leu Asp Ala
    2900                    2905                    2910

Phe Ser Leu His Thr Tyr Thr Pro His Glu Leu Thr Arg Val Ala
    2915                    2920                    2925

Ser Ala Leu Arg Lys Leu Gly Ala Pro Pro Leu Arg Ala Trp Lys
    2930                    2935                    2940

Ser Arg Ala Arg Ala Val Arg Ala Ser Leu Ile Ser Arg Gly Gly
    2945                    2950                    2955

Arg Ala Ala Val Cys Gly Arg Tyr Leu Phe Asn Trp Ala Val Lys
    2960                    2965                    2970

Thr Lys Leu Lys Leu Thr Pro Leu Pro Glu Ala Arg Leu Leu Asp
    2975                    2980                    2985

Leu Ser Ser Trp Phe Thr Val Gly Ala Gly Gly Gly Asp Ile Tyr
    2990                    2995                    3000

His Ser Val Ser Arg Ala Arg Pro Arg Leu Leu Leu Phe Gly Leu
    3005                    3010                    3015

Leu Leu Leu Phe Val Gly Val Gly Leu Phe Leu Leu Pro Ala Arg
    3020                    3025                    3030

## Description

In a first aspect, the present invention relates to new compounds based on diphenylpiperazine and diphenylpiperidine structures. In particular, the present invention provides new flunarizine derivatives having improved hepaci virus infection inhibitory activity. In a further aspect, the present invention relates to a pharmaceutical composition containing said compound as well as the use of said pharmaceutical composition and the compounds according to the present invention in preventing or treating hepaci virus infection, in particular, HCV virus infection, like HCV of genotype 2. Moreover, a method for determining effectiveness of prophylactic or therapeutic treatment of hepaci virus, like HCV infection as well as a method for determining the therapy regimen of an individual afflicted with hepaci virus infection including HCV infection is provided. Said method is based on determining the sequence or interfacial hydrophobicity of the hepaci virus E1 protein. This may include determining the presence of mutations at predetermined positions of the E1 sequence. Based on determining the interfacial hydrophobicity of the E1 protein, the sensitivity to a diphenylpiperazine or diphenylpiperidine based hepaci virus inhibitor as well as a phenothiazine and cycloheptenepiperidine based hepaci virus inhibitor can be determined. When the central hydrophobicity region is disrupted or the hydrophobicity is below zero applying the Wimley-White hydropathy plot, or the mutations at positions 290, 299, 301 and 310 of SEQ ID No. 1 are present, it is submitted that the sensitivity against said compounds is reduced. Hence, it is possible to determine the therapy regimen of an individual afflicted with hepaci virus infection or being a risk of being afflicted with hepaci virus infection, in particular HCV infection like HCV genotype 2 infection.

### Prior Art

Hepatitis C virus (HCV) is a highly variable, enveloped virus of the family F/*a-viviridae.* According to sequence analysis, viral isolates are classified into seven genotypes and 86 subtypes. HCV particles harbor a plus-strand RNA genome of positive polarity that encodes a polyprotein comprising structure proteins, the P7 ion channel and various non-structural proteins. Virus particles are composed of the core protein that encases the viral RNA, and of the envelope 1 protein (E1) and envelope 2 protein (E2). These are embedded in the viral lipid membrane. Virion-associated E1 and E2 glycoproteins coordinate interactions with cellular receptors, cell uptake and membrane interfusion which is triggered by the low pH in cellular endosomes.

Hepatitis C virus is a member of the hepaci virus genus. The Hepatitis C virus is also identified as the species hepaci virus C whereby humans are the only natural host. Additional hepaci virus are described, namely, hepaci virus A to N. Based on genetic differences between HCV isolates, the Hepatitis C virus species is classified into 6 genotypes (genotypes 1 to 7) with several subtypes within each genotype. Further differentiation in subtypes is done by separating these isolates in quasispecies. That is, a HCV of a genotype 2a is a HCV virus of genotype 2 with subtype a.

Chronic Hepatitis C virus infection is associated with severe liver diseases including hepatitis, cirrhosis and hepatocellular carcinoma. The WHO estimates that approximately 71 million individuals are chronically infected and at risk of developing disease. That is, HCV has a long persistence in humans without breakout of any symptoms of disease.

The licensing of multiple classes of directly acting antivirals (DAA) has in recent years revolutionized antiviral treatment. These drugs either target a viral NS3 protease, the NS5A phosphoprotein or the NS5B polymerase. Combination therapies involving these drugs are active against viruses from genotype 1 to 6 and reach curing rates of greater than 95 %. Although 95 % of cases may be cured, these treatments are costly and in some cases viral resistance can develop.

Resistance associated substitutions (RAS) have been observed for all classes of DAA and is associated with treatment failure. Although viral resistance and treatment failure occur infrequently, due to the relative short history of these new treatments, it is difficult to predict to which level salvage therapies including novel drug classes will be required in the future.

Cell entry inhibitors comprise part of HIV-1 combination therapies, and are in clinical development for treatment of Hepatitis B and Hepatitis delta virus infections. Also in case of HCV, cell entry inhibitors which either target viral components or cellular factors have emerged as interesting class of inhibitors, Colpitts C. C. et al., ACS infect Dis. 2017; 3:620-623. Preclinical studies have confirmed their efficacy *in vitro* and *in vivo* and have shown that combined with DAAs they prevent breakthrough of antiviral-resistant HCV. In the course of several independent screening campaigns involving various compound libraries including for instant the NRH clinical compound (NCC) collection or the library of pharmacologically active compounds (NOPAC 1280), a number of HCV entry inhibitors were discovered, see Pietschmann T., J Virol 2017; 91.

These HCV entry inhibitors have diverse but related chemical scaffolds including molecules from the group of diphenylpiperazines, diphenylpiperidines, phenothiazines, thioxantenes, and cycloheptenepiperidines. This group includes licensed drugs with neuroleptic or anti-histamine activity, like flunarizine and chlorcyclizine. That is, recently it has been reported that flunarizine as a clinically improved ion channel inhibitor and neuroleptic, inhibits HCV cell entry by preventing viral membrane fusion, see Perin, et al., 2016, Hepatology, 63, 49-62. It has been shown that fluphenazine, a phenothiazine, and pimozide, which both are structurally related to flunarizine, inhibit HCV infection by common mode of action. Further, it has been shown that these compounds specifically inhibit HCV entry and not infection by other enveloped viruses. Others reported that chlorcyclizine, a diphenylpiperazine and antihistamine that is structurally related to flunarizine, inhibits HCV infection, see He et al., Science Translation in Medicine 2015, 7, 282 RA 249. Antiviral activity in the nanomolar range and proven *in vivo* efficacy render some of these compounds attractive candidates for further development as HCV entry inhibitors. However, it remains unclear if these diverse compounds share their mode of action, if they target a common virus, and the susceptibility of virus against these inhibitors.

However, there is an ongoing need for new therapeutics for preventing and treating hepaci virus infection in a subject, in particular, for preventing and treating HCV infection overcoming known resistance of HCV inhibitors. Moreover, there is a need to address current and future problems associated with viral drug resistance to known drugs.

### Brief description of the present invention

The first aspect, the present invention relates to a compound of the general formula I 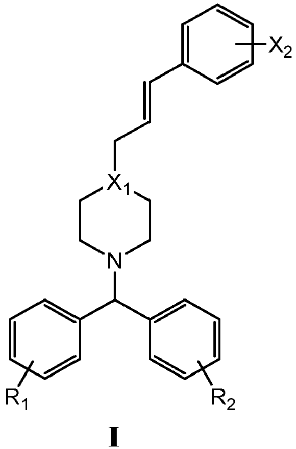
wherein R₁ and R₂ are independently from each other selected from a group of H, Halogen or a C₁ - C₄ alkyl group whereby at least one of R₁ and R₂ is a halogen, R₁ and/or R₂ may be present once, twice, three, four or five times at the aryl group;
X₁ is C or N;
X₂ is selected from a OR₃ group or a NR₄R₅ group wherein
R₃ is H or a C1 - C4 alkyl group, and wherein X₂ may be present once, twice, three, four or five times at the aryl group;
R₄ and R₅ are independently from each other selected from the group of H, N, C₁ - C₄ alkyl group, and (CH₂)ₙ - CR₆R₇R₈;
wherein R₆ and R₇ are independently from each other selected from H or a C₁ - C₄ alkyl group, and
R₈ is H, C₁ - C₄ alkyl group or a cyclopropanyl group;
n is 0 or 1;
or salts, solvates or hydrates thereof. It is preferred that X₂ is present in para-position.

The present inventors recognized that the compounds of the general formula I have improved activity as hepaci virus membrane fusion inhibitor, in particular, as HCV inhibitor. The activity of said compounds according to the present invention is superior over the activity of flunarizine as well as chlorcyclizine described in the art.

In a further aspect, the present invention provides a pharmaceutical composition containing the compounds according to the present invention. Further, the compound according to the present invention as well as the pharmaceutical composition according to the present invention is for use in preventing or treating hepaci virus infection. Hepaci virus infection include non-primate hepaci virus like non-primate hepaci virus from horse, rat, gorilla, rodent, bat and bovine. In an embodiment, the hepaci virus infection is a hepatitis C virus infection in particular, HCV of genotype 2 (HCV GT2).

In another aspect, the present invention provides the compound according to the present invention or a pharmaceutical composition according to the present invention in combination with a further anti hepaci virus inhibitor known in the art, in particular, direct antiviral agents targeting viral NS3 protease NS5A phosprotein or NS5B polymerase.

Additionally, the present invention relates to a method for determining effectiveness of prophylactic or therapeutic treatment of hepaci virus infection, like HCV infection, in particular, HCV type 2 infection, comprising:
- Determining the interfacial hydrophobicity of E1 of the hepaci virus, like E1 of the HCV strain, using the Wimley and White interfacial hydrophobicity scale;
- Determining the sensitivity to a diphenylpiperazine or diphenylpiperidine based hepaci virus inhibitor as well as phenothiazine and cycloheptenepiperidine based hepaci virus inhibitors, in particular, a compound according the present invention,
wherein a disrupted central hydrophobic region of the E1 protein or a hydrophobicity of the E1 protein lower than zero at the next following His residue, as has been determined with the Wimley-White hydropathy plot is indicative for reduced sensitivity to a diphenylpiperazine or diphenylpiperidine based hepaci virus inhibitor as well as phenothiazine and cycloheptenepiperidine based hepaci virus inhibitors.

In another aspect, the present invention relates to a method for determining the therapy regimen of an individual afflicted with hepaci virus infection or being at risk of being afflicted with hepaci virus infection, in particular, having or being at risk of HCV infection, comprising the step of
- Providing a sample obtained from said individual and determining the interfacial hydrophobicity of the E1 protein of the hepaci virus, like E1 of the HCV or its homolog from hepaci virus, in particular, using the Wimley-White hydropathy plot;
- Analyzing whether the central hydrophobicity region is disrupted or the hydrophobicity is below zero at the next following His residue, in particular, the hydrophobicity is below zero applying the Wimley-White hydrophathy plot;
- Determining the therapy regimen wherein no inhibitor of the E1 protein of hepaci virus, like of HCV or its homolog, in particular, a diphenylpiperazine or diphenylpiperidine based HCV inhibitor as well as phenothiazine and cycloheptenepiperidine based hepaci virus inhibitors is applied when the central hydrophobicity region is disturbed or the hydrophobicity is below zero at the next following His residue applying the Wimley-White hydropathy plot.

### Brief description of the drawings

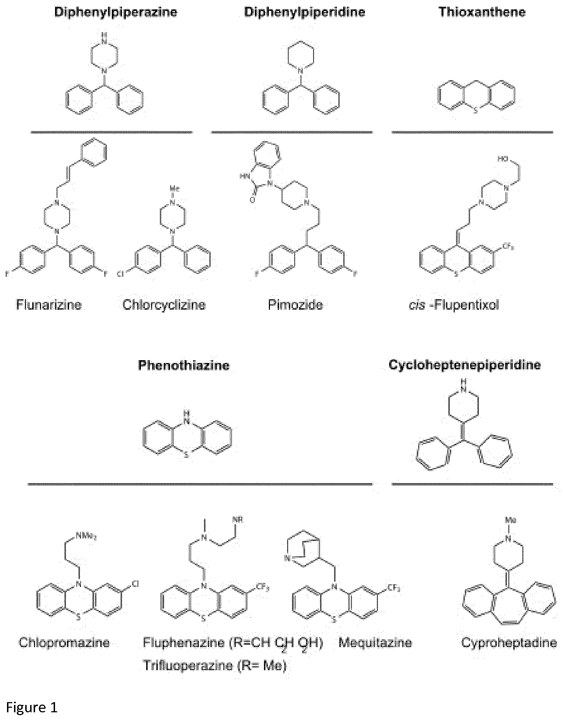

Fig. 1. Resistance mutations to flunarizine confer cross-resistance to structurally related HCV entry inhibitors. Chemical scaffolds of recently described HCV entry inhibitors of the diphenylpiperazine, diphenylpiperidine, thioxanthene, phenothiazine, and cycloheptenepiperidine families, Curcumin and BJ486K, two HCV entry inhibitors.

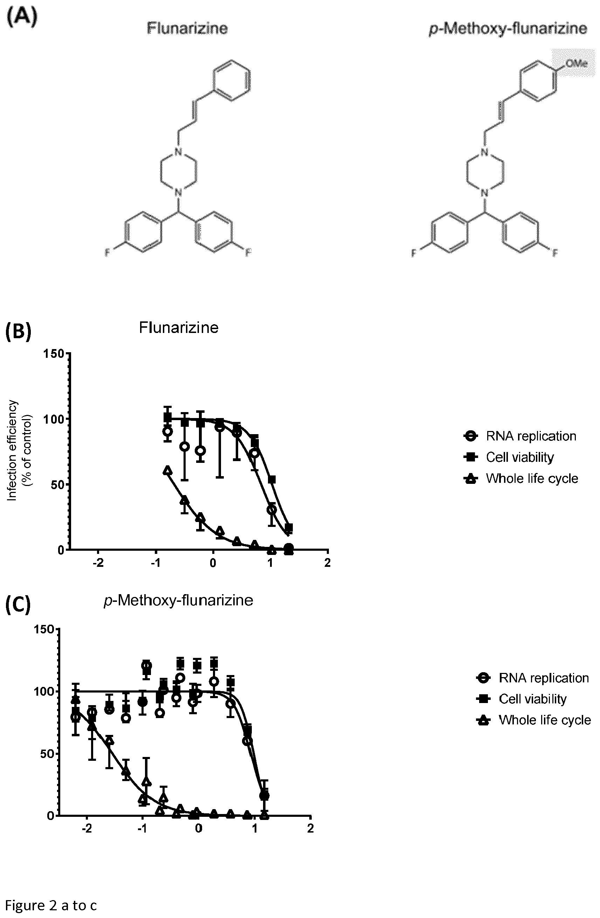

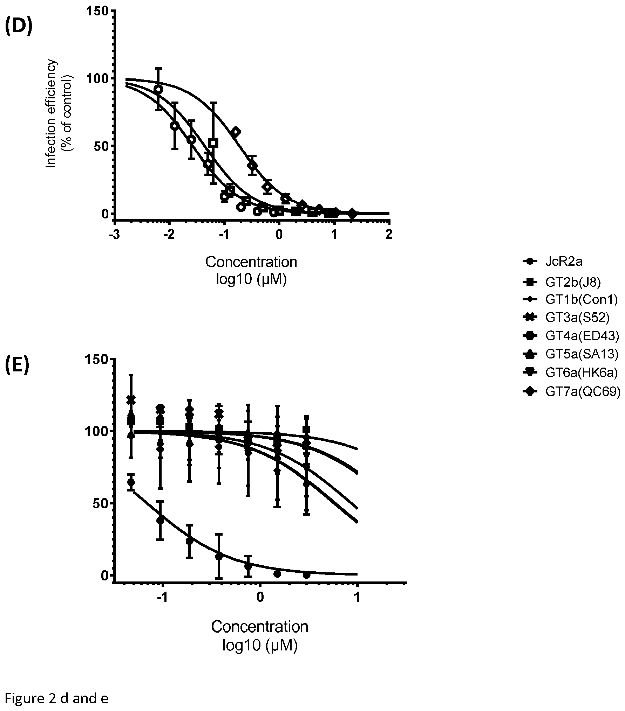

Fig. 2. p-methoxy-flunarizine exhibits enhanced antiviral activity and improved therapeutic index. A library of new flunarizine derivatives was synthesized and screened for antiviral activity in a viral whole life cycle assay (Table 1). (A) Structure of flunarizine, of p-methoxy-flunarizine and summary of structure activity relationship of flunarizine-related molecules. Whole life cycle JcR2a infection assay in presence of increasing doses of flunarizine (B) and p-methoxy-flunarizine (C), respectively. Cells are transfected with JcR2a and compounds are added 4h after transfection. HCV RNA replication is determined by quantifying renilla luciferase activity in transfected cells after 48h. Cytotoxicity of compounds is quantified at the same time point using cellular fluc expression. Culture fluid of the transfected cells is passed on to naïve cells where renilla luciferase activity is determined 48h later. (D) Comparison of antiviral activity of p-methoxy-flunarizine, chlorcyclizine and flunarizine as determined by the HCV whole life cycle infection assay (E) Antiviral activity of p-methoxy-flunarizine was examined by using given chimeric reporter viruses. Mean values of three biological replicates +/-SD are given.DMSO was used as vehicle control.

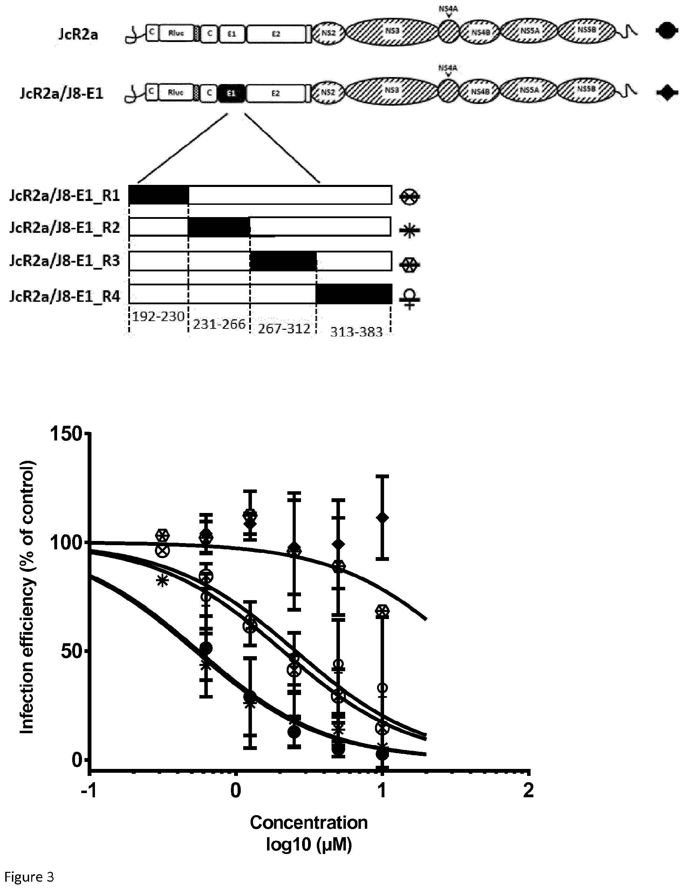

Fig. 3. Viral determinants within E1 control sensitivity to flunarizine. Susceptibility of chimeric JcR2a reporterviruses with J8-(GT2b) derived E1 regions. Infection is expressed relative to control infections in presence of solvent (DMSO). Mean values of three biological replicates +/- SD are given.

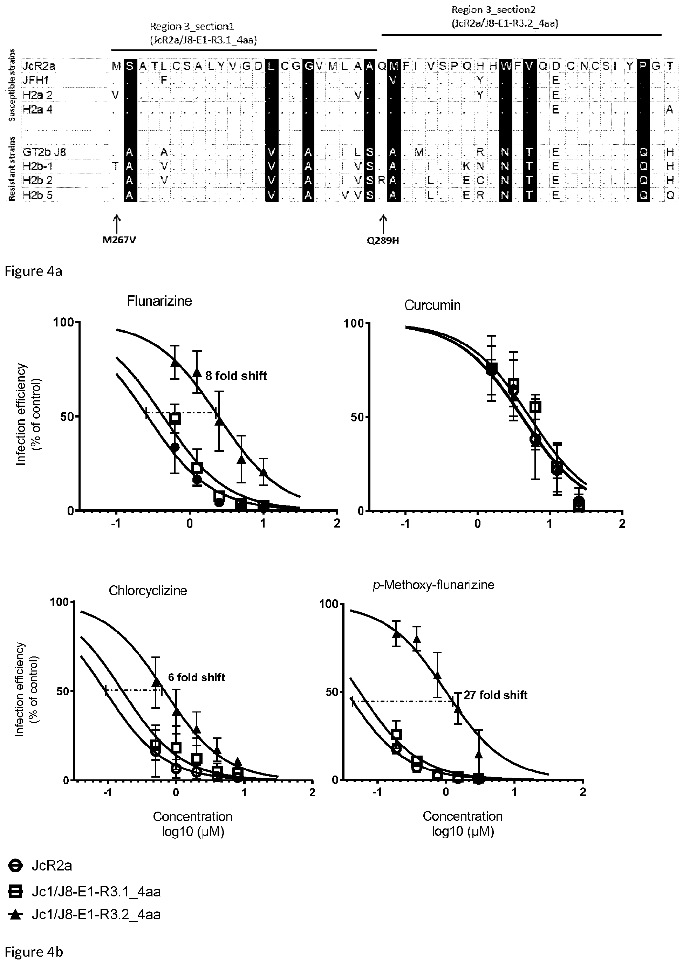

Fig. 4. Four conserved residues proximal to flunarizine resistance mutations and the putative fusion loop govern susceptibility to HCV membrane fusion inhibitors. (A) Sequence alignment of E1 protein region covering the putative fusion loop, flunarizine resistance mutations and region 3 of E1. Four GT2a-derived strains susceptible to flunarizine are given at the top, and four GT2b-derived isolates resistant to flunarizine are plotted below. Susceptibility of these strains to flunarizine was examined in Perin et al. Flunarizine resistance mutations are M267V and Q289H, fully conserved residues that distinguish sensitive and resistant strains are boxed. (B) Inhibition of JcR2a reporter viruses by given entry inhibitors. Infection is expressed relative to control infections in presence of solvent (DMSO). Mean values of three biological replicates +/-SD are given.

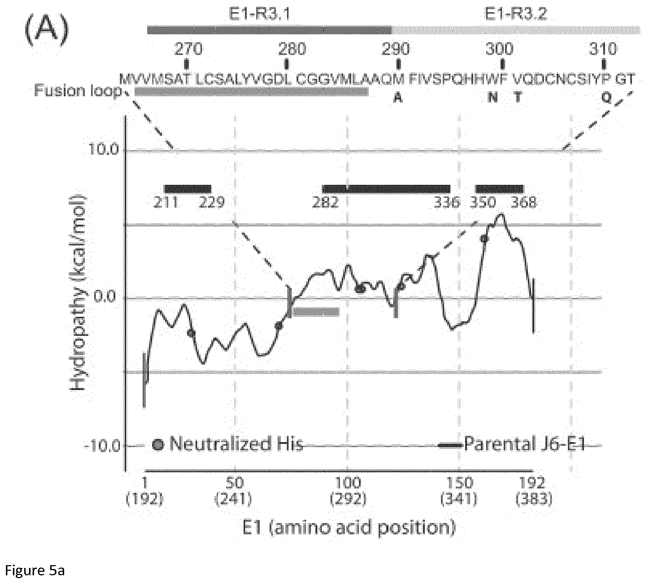

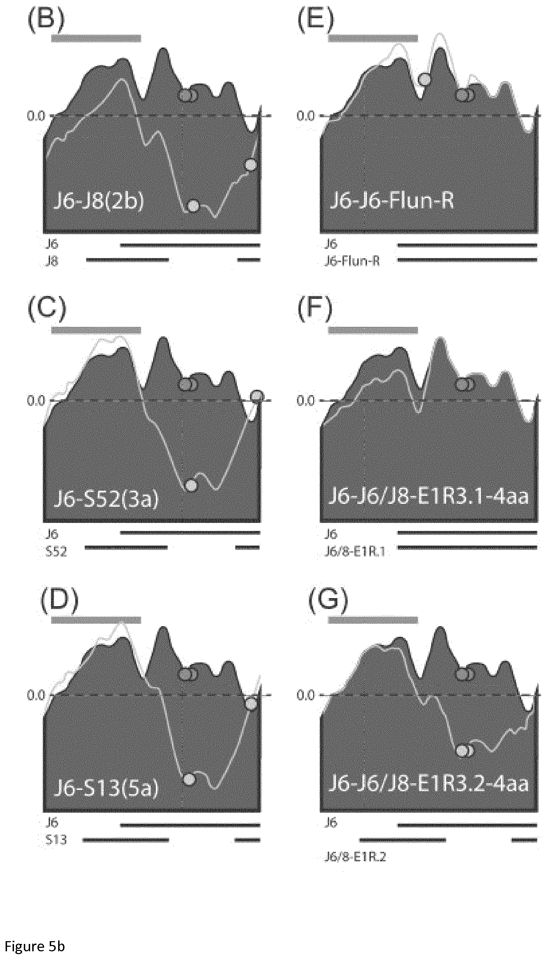

Fig. 5. Predicted hydrophaty of HCV E1 proteins with a focus on the central region encompassing the putative fusion loop and residues governing sensitivity to flunarizine. (A) Wimley-White hydropathy plot for the J6-E1 protein. Predicted hydrophobic regions are indicated by thick black bars on top of the smoothed predicted hydropathy (thin black line). The primary amino acid sequence of the central E1 region from residues 264 to 312 is provided above. The putative fusion loop is indicated by a bar (Aa 265-287), E1 region 3 section 1 (Aa 267-289) and section 2 (290-312) characterized in this study are indicated by bars. Residues conserved among flunarizine resistant HCV GT2b strains are plotted in bold face directly below the J6 sequence. Neutralized histidine residues are shown by circles. (B-G) Comparison of predicted hydropathy of central E1 region (residues 264-312) between J6-E1 and given alternative E1 proteins. Neutralized histidines are shown in circles given for J6 and for the alternative E1 proteins.

### Detailed description of the present invention

The present invention refers to a compound of general formula I 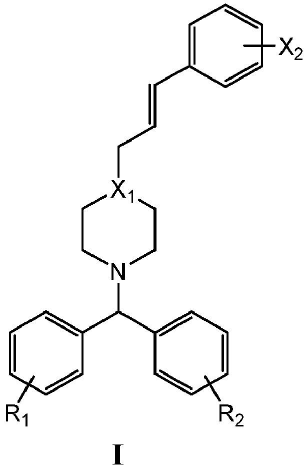
wherein R₁ and R₂ are independently from each other selected from a group of H, Halogen or a C₁ - C₄ alkyl group whereby at least one of R₁ and R₂ is a halogen, and R₁ and/or R₂ may be present once, twice, three, four or five times at the aryl group;
X₁ is C or N;
X₂ is selected from a OR₃ group or a NR₄R₅ group wherein
R₃ is H or a C1 - C4 alkyl group, and X₂ may be present once, twice, three, four or five times at the aryl group;
R₄ and R₅ are independently from each other selected from the group of H, N, C₁ - C₄ alkyl group, and (CH₂)ₙ - CR₆R₇R₈;
wherein R₆ and R₇ are independently from each other selected from H or a C₁ - C₄ alkyl group, and
R₈ is H, C₁ - C₄ alkyl group or a cyclopropanyl group;
n is 0 or 1;
or salts, solvates or hydrates thereof.

Preferably, X₂ is at least in para-position. In an embodiment, X₂ is present at the para-positon only.

The molecules and compounds according to the present invention targets the new drug target, E1, thus, representing an alternative to the known NS3, NS5a or NS5b inhibitors of hepaci virus, including hepatitis C. In particular, the compounds according to the present invention are new compounds having superior activity for inhibiting virus membrane fusion compared to known compounds including flunarizine or chlorcyclizine.

These compounds are particularly relevant and useful for treating subgroups of hepaci virus infected individuals, namely, individuals having an interfacial hydrophobicity of E1 of Zero or above using the Wimley and White interfacial hydrophobicity scale (White SH & Wimley WC (1999). Annu. Rev. Biophys. Biomol. Struc. 28:319-365). The compounds according to the present invention demonstrates a high-antiviral activity in a whole lifecycle compounds screening assay. In particular, further substituent at the para position to the allyl element modulate and increase the antiviral activity. Particularly, differently substituted amino substituent including a free amino group, triazoles demonstrate a high activity while changes at other positions, e. g. an additional methyl substituent at the alkene is detrimental to the activity. That is, the compounds according to the present invention being substituted at the para position of the phenyl group adjacent to the allyl element (a cinnamyl group) has beneficial effects on antiviral activity. This is also true for functional groups like an acid group.

The term "alkyl" refers to a saturated aliphatic hydrocarbon including straight chain and branched chain groups. The alkyl groups may be substituted or unsubstituted. Substitutions may also be themselves substituted. When substituted, the substituent group is preferably, but not limited to, C1-C3 alkyl, amino, cyano, halogen, C1-C3 alkoxy or hydroxyl.

As used herein, the term C₁ to C₄ alkyl group include a C₁ alkyl group, namely, methyl, an ethyl group (C₂ alkyl group), a propyl group and butyl group. A C₁ to C₄ alkyl group include straight or branched alkyl group, if possible.

In addition, the term halogen includes F, Cl, Br and I.

"Alkoxy" refers to the group -O-R with R being C1-C4 alkyl.

The term C1 to C4 alkoxy group refers to a methoxy, ethoxy, propoxy or butoxy group.

The term "salts" refers to any ionic form of a compound and one or more counter-ionic species (cations and/or anions). The term "salt" additionally includes zwitterionic compounds (i.e. a molecule containing one or more cationic and anionic species, e.g. zwitterionic amino acids). Counter ions present in a salt can include any cationic, anionic, or zwitterionic species. Examples of anions include, but are not limited to: chloride, bromide, iodide, nitrate, sulfate, bisulfate, sulfite, bisulfate, phosphate, acid phosphate, perchlorate, chlorate, chlorite, hypochlorite, periodate, iodate, iodite, hypoiodite, carbonate, bicarbonate, isonicotinate, acetate, trichloroacetate, trifluoroacetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, trifluormethansulfonate, ethanesulfonate, benzenesulfonate, p-toluenesulfonate, p-trifluoromethylbenzenesulfonate, hydroxide, aluminates and borates. Examples of cations include, but are not limited to: monovalent alkali, metal cations, such as lithium, sodium, potassium, and cesium, and divalent alkaline earth metals, such as beryllium, magnesium, calcium, strontium, and barium. Also covered by this term are transition metal cations, such as gold, silver, copper and zinc, as well as non-metal cations, such as ammonium salts.

As used herein, the term "pharmaceutically acceptable" is used to refer to those compounds, materials, compositions, and/or dosage forms which are suitable for use in contact with the tissues of human beings and animals without excessive toxicity, irritation, allergic response, or other problem or complication, commensurate with a reasonable benefit/risk ratio.

A "therapeutically effective dosage" of a compound is the amount that is required to inhibit hepaci virus, like HCV entry into cells in order to reduce the hepaci virus, like HCV infection rate to ≤ about 10%. In other words, a "therapeutically effective dosage" of the compound is the amount needed to cause an effect in vivo that ≥ about 90 % of infection will be reduced. It is known in the art that the therapeutically effective dosage of a drug depends on the route of administration.

The present invention also includes pharmaceutically acceptable salts of the compounds according to the present invention, e.g. being present in a pharmaceutical composition according to the present invention. As used herein "pharmaceutically acceptable salts" refers to derivatives of the compounds according to the present invention wherein the parent compound is modified by converting an existing acid or base moiety to its salt form. Examples of pharmaceutically acceptable salts include, but are not limited to, mineral or organic acid salts of basic residues such as amines; alkali or organic salts of acidic residues such as carboxylic acids, and the like. Generally, such salts can be prepared by reacting the free acid or base forms of the compounds with a stoichiometric amount of the appropriate base or acid in water or in an organic solvent, or in a mixture of the two; generally, non-aqueous media like ether, ethyl acetate, alcohols (e.g. methanol), ethanol, iso-propanol, or butanol) or acetonitrile are preferred. The compounds according to the present invention including salts, solvates or hydrates thereof can be prepared using known organic synthesis techniques and can be synthesized according to any of numerous possible synthetic routes.

In an embodiment of the present invention, the compound of general formula I is a compound wherein residue X₁ is N. That is, in an embodiment of the present invention, the compound of general formula I is a piperazine based compound. Alternatively, in case when X₁ is C, the compound according to the present invention is a piperidine based compound.

In another embodiment of the present invention, the halogen of R₁ and R₂ is F. In an embodiment of the present invention, at least one of R₁ and R₂ is F, in another embodiment, both of R₁ and R₂ are F. Other embodiments include the presence of at least two R₁ residues and/or two R₂ residues at the phenyl groups. However, in an embodiment, the halogen, in particular, F, representing R₁ and/or R₂ is in para position.

In another embodiment, the compound of general formula I according to the present invention is a compound wherein X₂ is a C₁ to C₄ alkoxy group, in particular, a methoxy group. Alternatively, the compound of general formula I is a compound wherein X₂ is a NR₄R₅ group wherein at least one of R₄ and R₅ is hydrogen.

Another embodiment identifies a compound of general formula I wherein NR₄R₅ is a group selected from NH₂, N₃, NH-CH(CH₂)₂, NH-C-tertbutyl, NH-ethylcyclopropyl. Other embodiments of the present invention includes compounds of general formula I wherein X₂ is anyone of the following structures

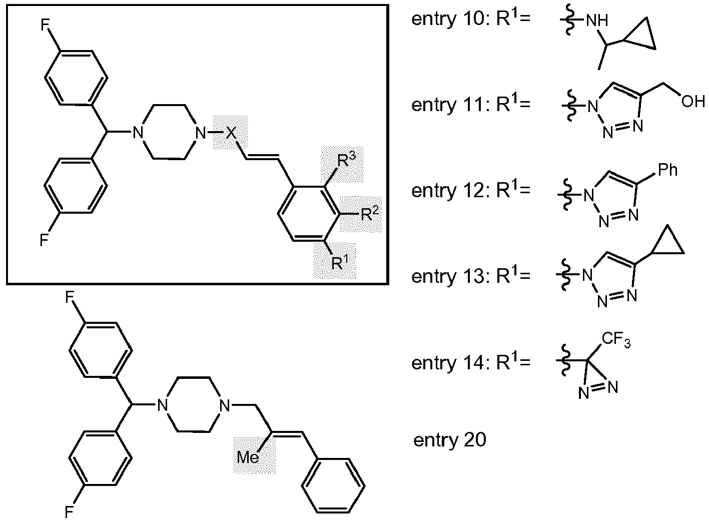

In the context of the present invention, the term "comprising", "comprises", "containing" or "contains" include the embodiments of "consisting of" or "consist".

In an embodiment of the present invention the compound of general Formula I is a compound of the following

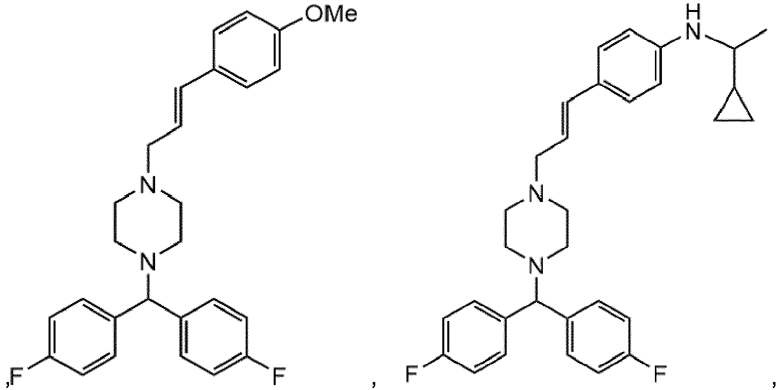

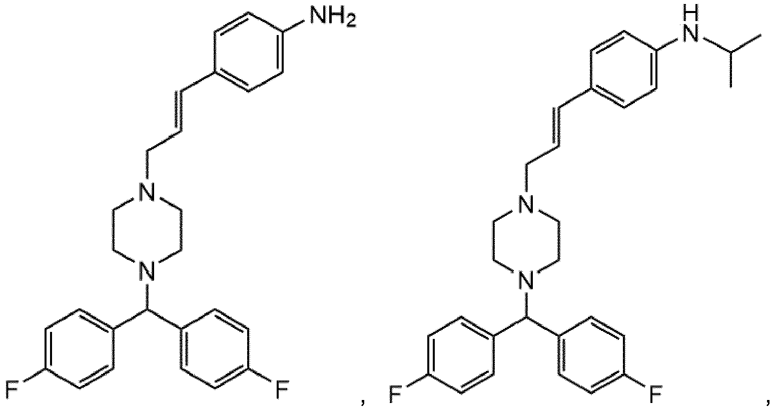

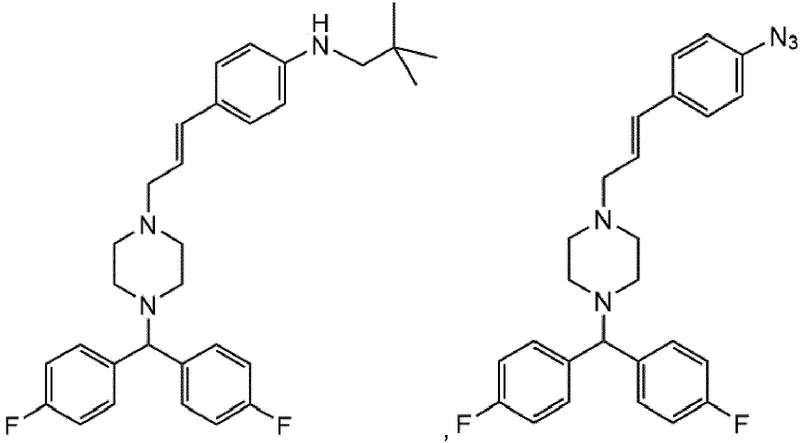

In another embodiment, the present invention relates to a pharmaceutical composition containing a compound of general Formula I according to the present invention. The pharmaceutical composition according to the present invention as well as the compound of general Formula I according to the present invention are useful for preventing and/or treating hepaci virus infection. Hepaci virus infection include non-primate hepaci virus including hepaci virus A, B, D to N of non-primate hepaci virus from horse, rat, gorilla, rodent, bat, or bovine. In an embodiment, the hepaci virus is Hepatitis C virus (HCV).

Compounds according to the present invention may be used in form of its pure compounds or of its salts thereof or in form of solvates, like hydrates. For example, the compounds according to the present invention may be administered in form a pharmaceutical acceptable salts thereof. The pharmaceutical composition according to the present invention may comprise further suitable diluents, excipients, or carriers. The pharmaceutical composition may be administered with a physiologically acceptable carrier to an individual. In a specific embodiment, the term "pharmaceutically accepted" means approved by regulatory agency or other generally recognized pharmacopoea for use in animals and, more particularly, in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly, for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skin milk, glycerol, propylene glycol, water, ethanol and the like. The pharmaceutical composition, if desired, can also contain minor amounts of wetting or emulsifying agent, or pH buffering agents. These compositions can take the form of solutions, suspensions, emulsion, tablets, pills, capsules, powders, sustained-release formulations, patches and the like. The compositions will contain a therapeutically effective amount of the compounds according to the present invention, preferably in purified form, together with a suitable amount of carrier so as to provide the form for proper administration to the individual. The formulation should suit the mode of administration. The route of administration of the compounds of the present invention depends on the formulation in use. The composition is formulated in accordance with routine procedures as the pharmaceutical composition adapted for intravenous or oral administration to individuals including human beings. Typically, composition for intravenous administrations or solutions in sterile isotonic aqueous buffer.

The term "administered" means administration of therapeutically effective dose or dosage of the pharmaceutical composition or the compounds according to the present invention. The methods are applicable to both human therapy and veterinary applications. The administration of the pharmaceutical composition can be done in a physiologically acceptable carrier as discussed above, including, but not limiting to, orally, subcutaneously, intravenously, intra-arterially, intraorally, intramedullary, intrathecally, intraventiculary, intranasally, intrabronchially, transdermally, intrarectally, intraperitoneally, intramuscularly, intrapulmonarily, vaginally, rectally, or intraoculary. Effective doses may be extrapolated from dose-response curves derived from *in vitro* animal model tests. As it is known in the art and described herein, adjustments for systemic versus locally delivery, age, body weight, generally health, sex, diet, time of administration, drug interaction and the severity of the condition may be necessary, and will be ascertainable with routine experimentation by those skilled in the art.

In an embodiment of the present invention, the compounds according to the present invention are the only pharmaceutical active agent comprised in said pharmaceutical composition. Moreover, in another embodiment of the present invention, the pharmaceutical composition according to the present invention may contain at least one further anti-hepaci virus inhibitor, like an anti-Hepatitis C virus inhibitor. The at least one further anti-hepaci virus inhibitor, like an anti-Hepatitis C virus inhibitor may be a DAA, like an inhibitor of the NS3 protease, the NS5A protein or the NS5B polymerase. Further, ribavirin may be a further active agent. In particular, the further anti-hepaci virus inhibitor, like anti-Hepatitis C virus inhibitor is at least one of sofosbuvir, dasabuvir, simeprevir (TMC435,) paritaprevir, grazoprevir (MK-5172), daclatasvir, ledipasvir, ombitasvir, velpatasvir, elbasvir (MK-8742). In case where more than one active agent is present in the pharmaceutical composition, the pharmaceutical composition may be adapted for allowing simultaneous, separate or sequential administration of the different active agents.

In addition, the present invention provides a method for the treatment or for the prophylaxis of hepaci virus infection, like Hepatitis C virus infection. The method comprises the step of administering a therapeutically effective amount of a compound according to the present invention. The skilled person is well aware of determining the effective amount, however, for the purpose of the present invention, a therapeutically effective dosage of the compound according to the present invention may preferably from about 1 to 2000 mg/d, preferably from about 10 to 1000 mg/d and further preferably from about 20 to 100 mg/d, which may be administered in one or multiple doses.

The administration in the method for the treatment or prophylaxis according to the present invention may be effected by any route of administration including oral, parenteral, such as subcutaneous intravenous, intramascular, intraperitoral, intrahecal, transdermal, transmucosal, subdural, nasal, local or topical via iontophoresis, sublingual, by inhalation spray, aerosol or rectally and the like in dosage in its formulations optionally comprising conventional pharmaceutical accepted excipients, diluents or carriers.

In an embodiment of the present invention, the compound according to the present invention or the pharmaceutical composition according to the present invention is for use in preventing or treating Hepatitis C virus, in particular, wherein the HCV is HCV of genotype 2. In particular, the compounds of general Formula I or the pharmaceutical composition according to the present invention are for use in treating or preventing hepaci virus infection, like preventing or treating Hepatitis C virus infection, in particular, wherein the HCV is HCV GT2 in individuals being resistant to therapeutic drugs against other targets, including NS3 protease, NS5A phosphoprotein or NS5B polymerase.

Namely, the compounds according to the present invention representing DAAs having the advantage of not being affected by drug resistance against the known DAA.

In another embodiment, the present invention relates to a method for determining effectiveness of prophylactic or therapeutic treatment of hepaci virus infection, like HCV infection, in particular, HCV type 2 infection, comprising:
- Determining the interfacial hydrophobicity of the E1 of the hepaci virus, like the E1 of the HCV strain, using the Wimley and White interfacial hydrophobicity scale;
- Determining the sensitivity to a diphenylpiperazine or diphenylpiperidine based hepaci virus inhibitor, as well as to the phenothiazine and cycloheptenepiperidine based hepaci virus inhibitors, in particular, a compound of any one of claims 1 to 7,
wherein a disrupted central hydrophobic region of the E1 protein or a hydrophobicity of the E1 protein lower than zero at the next downstream His residue as has been determined with the Wimley-White hydropathy plot is indicative for reduced sensitivity to a diphenylpiperazine or diphenylpiperidine based hepaci virus inhibitor as well as phenothiazine and cycloheptenepiperidine based hepaci virus inhibitors.

Lower than zero refers to the mext His residue downstream an d most proximal. That is, the level of predicted hydrophobicity of the most proximal downstream His and continuity of the central hydrophobic region predicts flunarizine sensitivity. As shown in the examples, HCV strains showing a continuous central hydrophobic region and also a hydrophobicity greater than 0 around the proximal His are sensitive to flunarizine. In contrast, strains with a disrupted central hydrophobic region and also hydrophobicity lower than 0 around the proximal His residue are resistant to flunarizine.

A further embodiment identifies a method for determining the therapy regimen of an individual afflicted with hepaci virus infection or being at risk of being afflicted with hepaci virus infection, in particular, having or being at risk of HCV infection, comprising the step of
- Providing a sample obtained from said individual and determining the interfacial hydrophobicity of the E1 protein of the hepaci virus, like the E1 of the HCV or its homolog, in particular, using the Wimley-White hydropathy plot;
- Analyzing whether the central hydrophobicity region is disrupted or the hydrophobicity is below zero, in particular, the hydrophobicity is below zero at the next downstream His residue applying the Wimley-White hydrophathy plot;
- Determining the therapy regimen wherein no inhibitor of the E1 protein of hepaci virus, like of HCV or its homolog, in particular, a diphenylpiperazine or diphenylpiperidine based hepaci virus inhibitor as well as to the phenothiazine and cycloheptenepiperidine based hepaci virus inhibitors is applied when the central hydrophobicity region is disturbed or the hydrophobicity is below zero at the next downstream His residue applying the Wimley-White hydropathy plot.

The present inventors recognized that susceptibility of fusion inhibitors of hepaci virus membrane fusion, in particular, Hepatitis C virus membrane fusion depends on the hydrophobicity of the E1 protein of the hepaci virus, like the HCV, in particular, the HCV genotype 2.

The E1 protein of genotype 2a is exemplified by strain J6 with accession number AF177036. The protein sequence of E1 of J6 is SEQ ID No. 1.

The E1 protein sequence of genotype 2B is SEQ ID No. 2. The present inventors recognized that the hydrophobic E1 region of the central region from aa265 to aa312 of SEQ ID No. 1, namely, the hydrophobicity region of the E1 protein including the region of aa290 to aa312 of SEQ ID No. 1 is important for susceptibility to fusion inhibitors. Namely, this central hydrophobic E1 region determines the pH range of Hepatitis C virus membrane fusion.

As used herein, the Wimley-White prediction of hydrophobicity within the central region refers to the region from amino acids 265 to 312.

It is recognized by the present inventors that mutations at positions 290, 299, 301 and 310 of SEQ ID No. 1 disturb or disrupt the hydrophobicity in said region. In particular, it has been recognized that mutations M290A, W299N, V301T and P310Q are predictive for disruption of central hydrophobicity as it is the case for HCV strains of genotype 2B.

Of course, other algorithms may be applied for determining hydrophobicity. The skilled person is well aware of suitable algorithms and programs.

The method according to the present invention allows identifying suitable new fusion inhibitors for treating and preventing infection with hepaci virus, in particular HCV. This is particularly true for infection with HCV genotype 2. That is, the surprising identification of relevant changes in the hydrophobicity region result in disrupting the hydrophobicity of the E1 in the central hydrophobicity region allows to search *in silico* for new inhibitors and lead compounds. Hence, the present invention provides screening methods for identifying suitable inhibitors of membrane fusion of hepaci virus, in particular HCV. In particular, the method according to the present invention allows to predict suitability of a compound as a fusion inhibitor and, thus, as an active agent in preventing or treating infection. This is particularly true for infection with HCV, like HCV GT2.

The present invention will be described further by way of examples without limiting the same:

### MATERIALS AND METHODS

### HCV infection assay

Huh-7.5 cells stably expressing firefly luciferase (Huh-7.5-fluc) were seeded in 12 or 96 well plates. The following day cells were inoculated with infectious viral particles in presence of the test compounds and incubated at 37°C. After 4 hours, the inoculum was aspirated, cells were washed with PBS, fresh medium was applied and cells were incubated at 37°C. *Renilla* and firefly luciferase activity were quantified 48 hours later to determine infection efficiency and cell viability, respectively.

### Fusion at the plasma membrane assay

The assay were conducted as previously described in Perin, see above. Briefly, Huh-7.5 cells were seeded in 6 well plates (3 x 10⁵ cell/well) and incubated at 37°C overnight (18 hours). Subsequently, cells were pre-treated with 5 nM concanamycin A (Con A) for 1 hour at 37°C (additional steps were carried out in presence of 5 nM Con A to block acidification of endosomes). Cells were inoculated with virus preparations at 4°C for 2 hours. Subsequently, cells were washed twice with PBS, fresh medium with or without test compounds was added and cells were incubated at 37°C for one hour. After this, cells were treated with pH 5.0 citric acid buffer for 5 minutes at 37°C in presence or absence of the test compounds. Cells were washed with PBS again, and fresh medium with or without test compounds was added and cells were incubated at 37°C for three hours. Finally, cells were washed with PBS, fresh medium without ConA and test compounds was added, and incubation of cells was continued at 37°C for 48 hours. After this, cells were lysed and *Renilla* luciferase activity, indicative of infection efficiency, was assessed.

### HCV whole life cycle compound screening assay

Flunarizine derivatives were screened for their antiviral activity in the HCV whole life cycle compound screening assay as previously described in Perin, see above. Briefly, Huh-7.5-fluc cells were transfected with genomic RNA of a GT2a *renilla* luciferase reporter construct JcR2a Reiss S, et al. Cell host & microbe 2011;9:32-45. Cells were seeded in 96 well plates and incubated at 37°C. After 4 hours, media containing, serially diluted compounds was added to the cells which were further incubated at 37°C. After 48 hours, media from the transfected cells was harvested and inoculated onto a new set of naïve target Huh-7.5-fluc cells which were incubated at 37°C for 48 hours. The transfected cells were lysed with water and the cells lysate was analyzed for *renilla* and firefly luciferase output to assess viral genome replication and cell viability respectively. The target cells were lysed after 48 hours to assess *renilla* luciferase reporter activity for the whole life cycle.
**Plasmids:** The full length Jc1 renilla luciferase reporter virus genome JcR2a was described before, Reiss S, et al. Cell host & microbe 2011;9:32-45. Similarly, the monocistronic renilla luciferase reporter virus genomes H77c/1a/R2a, J4/1b/R2a, JcR2a, J8/2b/R2a, S52/3a/R2a, ED43/4a/R2a, SA13/5a/R2a, HK6a/6a/R2a and QC69/7a/R2a were reported in a previous study, Haid S, et al. Gastroenterology 2012;143:213-U772. Novel chimeras and point mutants created for this study were cloned into the JcR2a backbone. Specifically, chimeras JcR2a/J8-E1, JcR2a/J8-E1_R1, JcR2a/J8-E1_R2, JcR2a/J8-E1-R3, JcR2a-E1_R4 carrying different portions of J8-derived E1 in the context of the JcR2a genome were generated. Moreover, JcR2a point mutant with four J8-derived residues of E1 subregion R3.1 (S268A, L280V, G283A, A288S) and R3.2 (M290A, W299N, V301T, P310Q) were created. Cloning strategies for these novel constructs can be obtained upon request.
**In vitro transcription and electroporation:** 10 µg of plasmid DNA was linearized by restriction digestion with an endonuclease Mlul (NEB) and purified using the QIAprep Spin Miniprep kit (QIAgen) according to manufacturer's instructions. The linearized DNA was in vitro transcribed and RNA was purified using NucleoSpin RNA Clean-up kit (Macherey Nagel) according to manufacturer's instructions. Cells counted to a final concentration of 1 x 107 cells/ml were ressuspended in Cytomix containing 2 mM ATP and 5 mM glutathione and transfected with respective RNA by electroporation. Transfected cells were immediately transferred to complete DMEM before seeding to dishes.
**Luciferase infection assay:** Huh-7.5-fluc cells seeded one day before in 12 or 96-well plates (5,3 x 104 cells/well; 1 x 104/well respectively) were infected with Jc1 with renilla luciferase for 4 h in presence of different concentrations of compounds. Viruses and compounds were removed, cells were washed with PBS and fresh medium was added. After 48 h, cells were washed with PBS and lysed with MilliQ water. Firefly luciferase activity was evaluated by adding 20 µL of cell lysates into assay buffer (25mM glycylglycine, 15 mM MgSO4, 4 mM EGTA, 1 mM DTT, 2 mM ATP, 15 mM K2PO4) and luciferin solution (200 µM luciferin, 25 mM glycylglycine, pH 8) and measured for 20 seconds in a luminometer (Centro XS3 LB960; Berthold). Renilla luciferase activity was measured by adding 20 µL of cell lysates into renilla luciferase substrate in PBS (1 µM of coelenterazin; P.J.K.) and measured for 2 seconds in a luminometer (Centro XS3 LB960; Berthold).
**Wimley White hydrophobicity analysis:** Analysis of E1 protein hydrophobicity was conducted using the Membrane Protein Explorer software package which is made available by the white laboratory (UC, Irvine, USA) under the URL (http://blanco.biomol.uci.edu/mpex/). Primary amino acid sequence of full length E1 coding sequences of given strains were used for prediction selecting interfacial hydrophobicity for the analysis. Smoothed hydropathy was plotted, neutralized His residues and predicted hydrophobic segments were include as well.
**Statistical Analyses:** Data were analyzed using GraphPad Prism version 7.04 or version for Windows (GraphPad Software, La Jolla California USA, www.graphpad.com). IC50 values were calculated using the nonlinear regression model implemented in GraphPad with the least squares fitting method and fixed upper and lower plateaus. Differences in normalized mean infection efficiencies were tested for significance using ordinary two-way ANOVA followed by Dunnett's multiple comparison tests. P-values below the level of 0.05 were considered significant (*p < 0.05, **p < 0.01, ***p < 0.001, ****p < 0.0001).
**Virtual screening to identify HCV membrane fusion inhibitors:** Computational studies were undertaken to identify compounds similar to flunarizine and chlorcyclizine, which exhibit antiviral activity by inhibiting Hepatitis C virus (HCV) cell entry. The structure of N-terminal domain of HCV glycoprotein E1 was obtained from PDB 4UOI. The structure was minimized using forcefield Amber10:EHT of Molecular operating environment (MOE, chemical computing group), Molecular Operating Environment (MOE). Chemical Computing Group Inc., 1010 Sherbooke St. West, Suite #910, Montreal, QC, Canada, H3A 2R7, 2013.; 2013.08. Small molecule databases (Approx. 7-8 million small molecules) were screened for those small molecules, which resemble chemically to flunarizine or chlorcyclizine. Resulting small molecules were docked on E1, and were ranked on the basis of binding energy calculations after Molecular dynamics simulations. Once a compound was found active experimentally, further computational calculations involved sub-structure similarity search using fingerprints followed by molecular docking. Details of the methods used are as follows-
**Docking:** This was a blind docking simulation where entire PDB 4UOI was considered possible binding site. The side chains were kept free to move during force field refinement. Alpha PMI is the placement method used with default settings (sample per conformation = 10, maximum poses = 250). London dG rescoring was used with Alpha PMI placement. Termination criteria for force field refinement were set as gradient = 0.001 and iterations = 500.
**Molecular dynamics simulations:** Top scoring poses from docking were retained for molecular dynamics simulations using amber12 software. We allowed the Leap module of Amber, et al. Journal of computational chemistry 2005;26:1668-1688, to add missing hydrogen atoms and heavy atoms using the Amber force field (ff10) parameters, Lindorff-Larsen K, et al. Proteins 2010;78:1950-1958. To neutralize the charge of the system, we added sodium/chloride ions. The model was immersed in a truncated cubical shell of TIP3P water. A time step of 2 fs and a direct-space non-bonded cutoff of 10 A ° were used. After the protein preparation, all systems were minimized to remove the steric clashes that occurred. The systems were then gradually heated to 300 K over a period of 50 ps and then maintained in the isothermal-isobaric ensemble (NPT) at a target temperature of 300 K and a target pressure of 1 bar using a Langevin thermostat and a Berendsen barostat with a collision frequency of 2 ps and a pressure relaxation time of 1 ps, respectively. We constrained hydrogen bonds using the SHAKE algorithm, Ryckaert J-PC, G.; Berendsen, H.J.C. Numerical integration of the cartesian equations of motion of a system with constraints: Molecular dynamics of n-alkanes. J Comput Phys 1977;23:327-341. We have used the velocity-Verlet algorithm (default algorithm for the Amber MD package) for MD simulations. Particle mesh Ewald (PME) procedure was used to treat long-range electrostatic interactions using default parameters, Darden TY, D.; Pedersen, L. Particle mesh Ewald-an N-log(N) method for Ewald sums in large systems. J Chem Phys 1993;98:10089-10092. After bringing the systems to our suitable temperature and pressure of 300 K and 1 bar, respectively and equilibrating the system for 500 ps, the production run was continued for 20 ns in the isothermal- isobaric ensemble at the target temperature of 300 K and target pressure of 1 bar using the same Langevin thermostat and Berendsen barostat. The structures in the trajectories were collected at 10 ps intervals. The analysis of trajectories was performed with the Ptraj module of Amber.

### RESULTS

### Flunarizine resistance mutations confer cross-resistance to diphenyl piperazine, diphenyl piperidine, phenothiazine, thioxanthene and cycloheptene piperidine HCV entry inhibitors

Recently, it has been identified flunarizine, chlorcyclizine (CCZ), pimozide, chlorpromazine, fluphenazine, trifluoperazine, mequitazine, cis-flupentixol and cyproheptadine, which are from the diphenyl piperazine, diephenyl piperidine, phenothiazine, thioxanthene and cycloheptene piperidine families, respectively, as potent HCV entry inhibitors (Fig. 1). Passaging of an HCV genotype 2a virus (Jc1) in the presence of flunarizine yielded a combination of three mutations that map to the E1 and E2 proteins and which confer a ca. 50-fold resistance to flunarizine, Perin see above. These mutations also conferred cross-resitance to pimozide, fluphenazine, and trifluoperazine. To explore if susceptibility to other phenothiazine-, thioxanthene- or cycloheptene piperidine-derived entry inhibitors was also affected by these residues, we infected cells in the presence of increasing doses of these compounds with either the parental Jc1 renilla luciferase virus (JcR2a) or the flunarizine-resistant variant carrying four mutations (M267V and Q289H in E1 and M405T in E2 and I757T in p7; JcR2a-Flun-R which is J6-Flun R) (data not shown). Note that among these four mutations only the three changes in E1 and E2 contribute to flunarizine resistance. Curcumin, a structurally unrelated HCV entry inhibitor, was used as control (Fig. 1). As expected, all compounds dose-dependently inhibited JcR2a infection. Except for curcumin, which equally inhibited JcR2a and the flunarizine resistant Jc1 variant JcR2a-Flun-R, all other compounds were much less active against JcR2a-Flun-R (change in IC50 18 to 275-fold). These results obtained herein indicate that M267V, Q289H and M405T mutations confer cross-resistance to diphenyl piperazine, diphenylpiperidine, phenothiazine, thioxanthene and cyloheptene piperidine based HCV entry inhibitors and suggest that these molecules inhibit HCV cell entry through a common mechanism.

### Diphenyl piperazines, diphenyl piperidines, phenothiazines, and thioxanthenes inhibit low pH-triggered HCV cell membrane fusion

HCV infects liver cells by way of virus-cell membrane fusion, which is essential to deliver the viral genomic RNA into hepatocytes for initiation of infection. Fusion occurs after the virus has interacted with receptors and is internalized into endosomes. These receptor interactions prime the E1/E2 proteins to respond to the acidified pH in endosomes with conformational changes that mediate virus-host cell membrane fusion. When acidification of endosomes is inhibited - for instance by treatment with Concanamycin A that inactivates endosomal ATPases needed for acidification of these organelles - HCV infection is ablated. Under these circumstances infection can be rescued by briefly washing virus exposed cells with a low pH buffer that triggers virus-membrane fusion and allows infection. Using this setup we recently provided evidence that flunarizine specifically inhibits HCV membrane fusion since application of this compound only during the 5 minute low pH wash was sufficient for its full antiviral activity (data not shown). Here we compared the activity of a panel of HCV entry inhibitors in this membrane fusion assays. Compounds tested included a flunarizine-related diphenyl piperazine (chlorcyclizine), a diphenyl piperidine (pimozide), a phenothiazine (mequitazine), and a thioxanthene (cis-flupentixol). In addition we used BJ486K which is flavonoid that is structurally unrelated to flunarizine (Fig. 1) and likely inhibits HCV entry by a different mechanism. In line with this assumption, and in contrast to flunarizine, BJ486K did not inhibit HCV infection when administered only during the low pH washing step (Fig. 2). In contrast, all tested flunarizine-related compounds from the family of diphenyl piperazines, diphenyl piperidines, phenothiazines, and thioxanthenes inhibited HCV infection when added only during the low pH wash. This finding supported the conclusion that these related compounds selectively inhibit low pH-triggered HCV cell membrane fusion.

### Diphenyl piperidines preferentially inhibit of HCV GT2 membrane fusion

Among all tested compounds, flunarizine and chlorcyclizine emerged as most potent HCV membrane fusion inhibitors. Therefore, we focused our analysis on these two molecules. Previously we observed that flunarizine preferentially inhibits HCV GT2 viruses including viral strains from subtypes 2a, 2d, 2e, 2k, 2m and 2q and that it was less active against other viral genotypes, see Perin. To explore if cholorcyclizine exhibits a similar preference for GT2 over other HCV genotypes we tested inhibition of HCV strains Con1 (GT1b), J8 (GT2b), S52 (GT3a), ED43 (GT4a), SA13 (GT5a), HK6a (GT6a) and QC69 (GT7a). In contrast to curcumin which was similarly active against all tested viruses, both flunarzine and cholorcyclizine preferentially inhibited Jc1, a G2a virus chimera, and they were less active against a GT2b strain (J8) and all other strains from non-GT2 genotypes tested. Thus, both flunarizine and chlorcyclizine exhibit a similar strain-dependent antiviral activity suggesting that they inhibit HCV membrane fusion through a common mechanism, possibly involving a similar viral target site.

### Structure-activity-relationship (SAR)-studies with new flunarizine derivatives

We synthesized a series of novel flunarizine derivatives (Table 1). The design of this compound library focused on structural changes in the allylic element and the adjacent arene substituent. Therefore no structural changes in the piperazine ring and the bis(4-fluorophenyl)methane unit. All derivatives were prepared from 4,4'-difluorbenzophenone according to the synthetic protocol reported in reference, Weiwer M, et al. Bioorg Med Chem Lett 2012;22:1822-1826. With this compound library in hand we tested for their antiviral activity in a whole life cycle compound screening assay as described in Perin, et al. showing that thirty exhibited antiviral activity against the Jc1 reporter virus (Table 1). An additional methyl substituent at the alkene is also not beneficial (entry 20). Several additional substituents at the phenyl ring, particularly in the para position to the allyl element modulate and in selective cases increased the antiviral activity. Particularly, differently substituted amino substituents including the free amino group (entries 3, 6. 7 and 10), triazoles (entries 11-13) need to be mentioned. Noteworthy, substituents that can serve in photo affinity labeling studies (entries 4 and 14) only lead to a moderate loss of activity. Methoxy substituents located in the para and meta positions were found to be beneficial (entries 2 and 19). *p*-Methoxy-flunarizine (entry 2) exhibited an IC₅₀ of 28 nM in the whole life cycle infection assay while maintaining the typical preference for JcR2a (Fig. 2 and table 1). Cell viability of *p*-methoxy-flunarizine treated cells was reduced by 50% at a dose of 9.6 µM. Thus, *p-*methoxy-flunarizine emerged as most powerful compound with an IC50 ca. 2-fold lower than chlorcyclizine and ca. 8-fold lower than flunarizine (entry 1) and a therapeutic index (IC50/CC50) of 335 which is improved by 1,8 and 8,8-fold relative to chlorcyclizine and flunarizine, respectively.

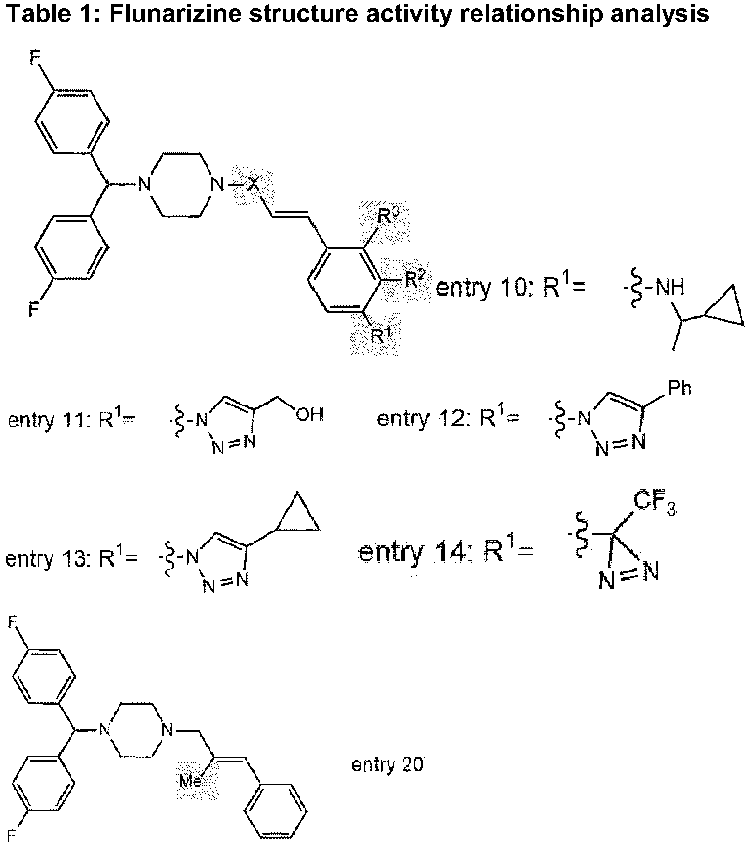

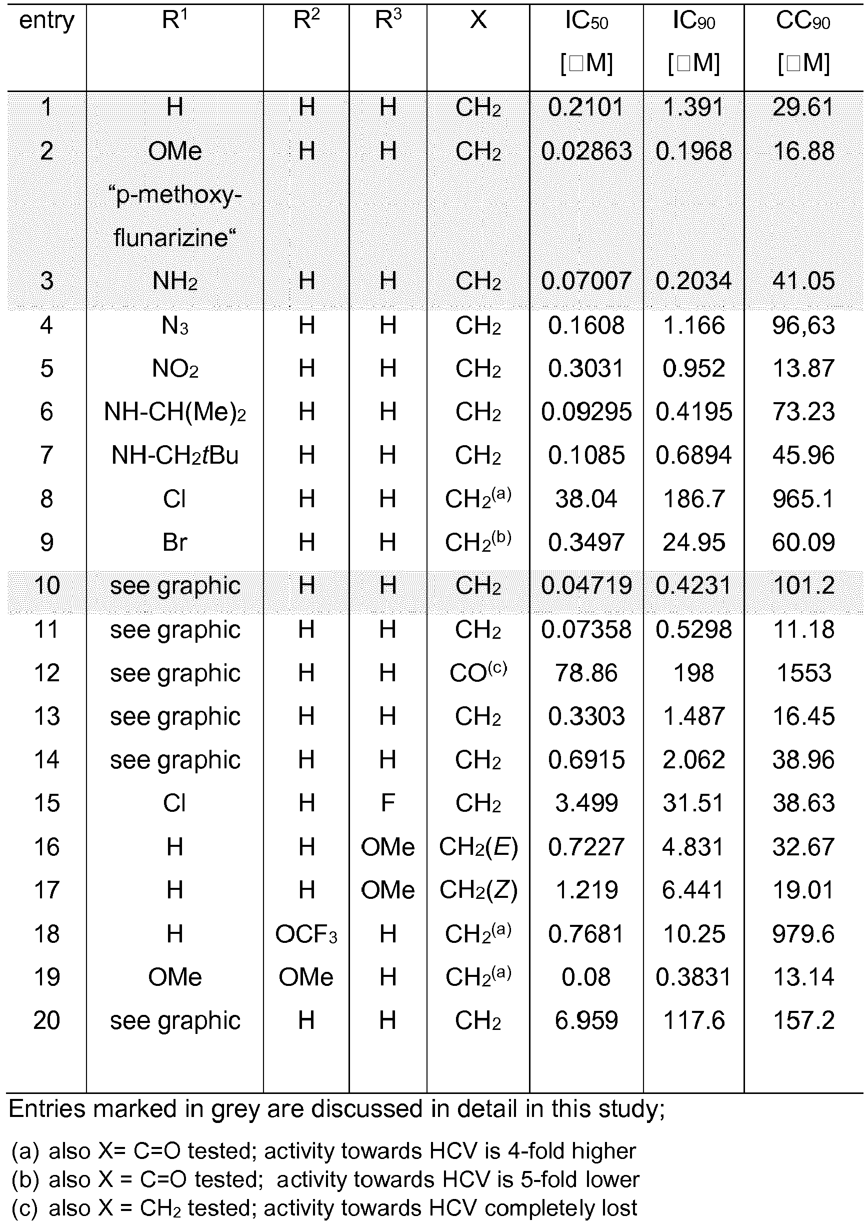

### Viral determinants of susceptibility to HCV membrane fusion inhibitors map to four residues in E1

p-Methoxyflunarizine derivative, flunarizine and chlorcyclizine, which emerged as potent representatives of HCV membrane fusion inhibitors, share a preference for inhibiting JcR2a over other HCV strains. While most GT2 strains are susceptible to flunarizine, J8 (GT2b) exhibits pronounced resistance to flunarizine, chlorcyclizine and p-methoxy-flunarizine (Fig.2). Thus, to map viral determinants responsible for sensitivity to these HCV membrane fusion inhibitors, we took advantage of this difference and created chimeras between the susceptible J6 (GT2a) and the resistant J8 (GT2b) strain. Replacement of Jc1 reporter virus glycoproteins E1 by J8-derived E1 rendered chimeric viruses resistant to flunarizine showing that primarily E1 determines susceptibility to this drug. Of note, replacement of Jc1-E1 for J8-E1 did not affect virus RNA replication and release of HCV particles as evidenced by comparable accumulation of luciferase reporter gene activity in lysates of transfected cells and by similar accumulation of released core protein. However, virus infectivity was much decreased suggesting that exchange of E1 selectively impaired cell entry. To compensate for this, virus stocks of the Jc1R2a/J8-E1 chimera were concentrated by ultrafiltration and virus stocks with luciferase transduction efficiency comparable to parental Jc1 were used for the drug resistance testing. Next we created Jc1 chimeras where only specific regions of E1 were replaced by the homologous J8 region. This did not affect RNA replication and virus release but infectivity of the chimeras was partially reduced and was again compensated for by using concentrated virus preparations. While Jc1 chimeras carrying the N-terminal E1 region of J8 (GT2b) (R1; Aa 192-230) or the most C-terminal region (R4; 313-383) displayed partial resistance to flunarizine, a chimera with the internal segments of E1 (R2; 231-266) remained fully susceptible (Fig. 3). In contrast, chimera Jc1/J8-E1_R3 (R3; Aa 267-312) exhibited a resistance profile comparable to Jc1 encoding the flunarizine resistance mutations or to Jc1 encoding the entire E1 protein of J8 (Fig. 3). Notably, two of the three observed flunarizine resistance mutations map to E1 region 3 (M267Y and Q289H; Fig. 3 and 4A) and this portion of E1 encompasses a putative fusion loop.

To find out which amino acids in this region are critical for conferring susceptibility to HCV membrane fusion inhibitors, we prepared a sequence alignment between four susceptible GT2a strains and four resistant GT2b isolates. We identified eight amino acids which are fully conserved among the sensitive strains and where the resistant strains encode a divergent residue that was fully conserved among the resistant strains. Thus, two additional JcR2a constructs were created encoding either these four N-terminal (i.e. S268A, L280V, G283A, A288S) or these four C-terminal (M290A, W299N, V301T, P310Q) residues of the GT2b strains instead of the cognate GT2a-typical residues. These mutant viruses exhibited RNA replication, virus release and infectivity comparable to parental JcR2a. When we analyzed sensitivity of these virus mutants to entry inhibitors, JcR2a/J8-E1-R3.1_4aa displayed comparable sensitivity to all tested inhibitors like parental JcR2a. In contrast, JcR2a/J8-E1-R3.2_4aa was much more resistant to flunarizine, chlorcyclizine, and p-methoxy-flunarizine (8-, 6-, 27-fold, respectively) but not to curcumin. Thus, four polymorphic residues encoded in the E1 region 291 to 312 (M290A, W299N, V301T and P310Q) determine susceptibility of HCV GT2a and 2b isolates to these HCV membrane fusion inhibitors.

### Susceptibility of HCV to membrane fusion inhibitors correlates with their pH requirements of fusion triggering

Many enveloped viruses initiate membrane fusion upon triggering of their fusion proteins in cellular compartments with low pH. Abundant protons in these acidic compartments mediate protonation of viral fusion proteins which in turn triggers conformational changes that bring viral and host membrane into close proximity allowing for injection of viral hydrophobic fusion peptides or fusion loops into the host cell membrane. This process also releases energy that is needed to overcome the energy barrier to virus-cell membrane fusion.

To test if sensitivity of HCV to flunarizine correlates with pH requirements of fusion protein triggering, we used a plasma membrane fusion assay (as described in Perin et al) and washed cell surface-bound HCV particles with buffers ranging from pH5 to pH7. Virus fusion and infection through the natural route of acidified endosomes was prevented by administration of ConA, thus allowing for quantification of virus fusion triggering and infection under defined pH conditions. In line with previously published studies describing the HCV pH requirements, Haid S, et al. JBiolChem 2009, pH 5 efficiently triggered infection of all tested viruses whereas pH 7 did not. However, the tested viruses exhibited significantly different responsiveness to pH values between 5 and 7. Flunarizine sensitive JcR2a exhibited the most stringent pH requirement and efficiently infected cells only when fusion was triggered with a pH of 5.0 (data not shown). In contrast, viruses that exhibited natural resistance to flunarizine (Gt2b, GT3a and GT5a) accepted a significantly broader range of pH including values approaching more neutral conditions. Therefore, natural resistance of HCV to membrane fusion inhibitors correlated with more relaxed requirements of HCV for pH-dependent induction of membrane fusion.

### Resistance mutations to membrane fusion inhibitors relax pH requirement for HCV fusion

Notably, Jc1 carrying the three resistance mutations to flunarizine also displayed relaxed pH requirements compared to parental Jc1. Moreover, single E1 mutations associated with flunarizine resistance (M267V and Q289H; extended the viral responsiveness towards more neutral pH. This phenotype was not further boosted by combination of both of these changes. Finally, the JcR2a variants encoding the four E1/R3.2_4aa residues conserved among naturally resistant Gt2b viruses instead of the cognate 2a-typical residues (M290A, W299N, V301T, P310Q) also fused upon triggering with more neutral pH compared to parental JcR2a and compared to the JcR2a variant encoding the four unique GT2b residues of E1/R3.1_4aa (S268A, L280V, G283A, A288S). Thus, single or combined point mutations that conferred resistance to flunarizine at the same time conferred a more relaxed responsiveness to fusion triggering.

### Resistance to flunarizine, and relaxed pH requirements for fusion correlate with modified hydrophobicity in a central E1 region proximal to the fusion loop

To investigate E1 protein features that may modify sensitivity to membrane fusion inhibitors and pH-dependent membrane fusion, we examined the interfacial hydrophobicitiy of HCV E1 from multiple strains using the Wimley and White interfacial hydrophobicity scale, Wimley WC, White SH. Experimentally determined hydrophobicity scale for proteins at membrane interfaces. Nat Struct Biol 1996;3:842-848, (Fig. 5). This scale provides a basis for understanding membrane protein folding and membrane insertion. Figure 5A displays the Wimley-White hydropathy plot for the J6-E1 protein and highlights changes in predicted hydropathy in the central E1 region which encompasses the putative fusion loop and residues controlling sensitivity to flunarizine and pH-dependent membrane fusion. J6 E1 encodes two histidine (His) residues (Aa 297, 298) within a large, continuous hydrophobic region downstream of the predicted fusion loop (Fig. 5A). His has a pKₐ of ca. 6-7 is the only amino acid whose protonation state changes in a pH range where viral membrane fusion is commonly activated. His is typically uncharged at neutral pH (prediction in Fig. 5A. However, lowering of pH leads to protonation of His, rendering it positively charged. This change can trigger conformational changes by repelling His away from positively charged residues towards negatively charged amino acids where it can engage novel salt bridges. Notably the local environment of His influences its pKₐ value and in turn the threshold for protonation and membrane fusion. All HCV strains analyzed here (Fig. 5 and Table 2) encode at least one His closely downstream of the putative fusion loop (Fig. 5).

**Table 2: Correlation between central E1 hydrophobicity, flunarizine sensitivity and pH range of HCV membrane fusion**

| **Strain name** | **Gen o-type** | **Accession number** | ***Central hydrophobicity** | ****Proximal His** | **^{§}Flunarizine sensitivity** | **^{$}pH requirement** | **^{$$}Predicted flunarizine sensitivity** |
|---|---|---|---|---|---|---|---|
| ^{#}J6 | 2a | AF177036 | continuous | >0 | yes | narrow | High |
| JFH1 | 2a | AB047639 | continuous | >0 | yes | n.d. | High |
| ^{##}H2a -2 | 2a | KM361733.1 | continuous | >0 | yes? | n.d. | High |
| H2a-4 | 2a | KM361735.1 | disrupted | Ca. 0 | yes? | n.d. | Unclear |
| J8 | 2b | JQ745651 | disrupted | <0 | no | broad | Low |
| H2b-1 | 2b | KM361727.1 | disrupted | <0 | no | n.d. | Low |
| H2b-2 | 2b | KM361728.1 | disrupted | <0 | no | n.d. | Low |
| H2b-5 | 2b | KM361731.1 | disrupted | <0 | no | n.d. | Low |
| 2d | 2d | JF735114.1 | continuous | <0 | yes? | n.d. | Unclear |
| 2k | 2k | AB031663.1 | disrupted | <0 | yes? | n.d. | Low |
| 2m | 2m | JX227967 | disrupted | <0 | yes? | n.d. | Low |
| 2q | 2q | FN666429 | disrupted | <0 | yes? | n.d. | Low |
| 2r | 2r | JF735115 | continuous | <0 | yes? | n.d. | Unclear |
| ^{###}H7 7 | 1a | NC_038882 | continuous | <0 | no | n.d. | Unclear |
| Con1 | 1b | AJ238799.1 | disrupted | <0 | no | n.d. | Low |
| SA52 | 3a | EU204645.1 | disrupted | <0 | no | broad | Low |
| ED43 | 4a | NC_009825 | disrupted | <0 | no | n.d. | Low |
| SA13 | 5a | FJ393024 | disrupted | <0 | no | broad | Low |
| HK6a | 6a | MG717928.1 | continuous | <0 | no | n.d. | Unclear |
| QC69 | 7a | EF108306.2 | disrupted | <0 | no | n.d. | Low |

| | | | | | | | |
|---|---|---|---|---|---|---|---|
| *Wimley-White prediction of hydrophobicitiy within the central region from Aa 265-312 ^{**}Wimley-White prediction of hydrophobicitiy directly surrounding the His residue most proximal downstream of the putative fusion loop (Aa 265-287) ^{§} Flunarizine sensitivity with: yes, when IC50 is <1µM; no, when IC50 >1µM; yes?, when antiviral activity was reported but IC50 was not determined ^{$} pH requirement to initiate membrane fusion and infection in the plasma membrane fusion assay. Narrow, when no significant difference to J6 reference; broad, when at any of the tested pH values there is significantly more fusion and infection observed ^{$$}Predicted flunarizine sensitivity: High, when continuous hydrophobicity and proximal His residue with hydrophobicity >0; Low, when hydrophobicity disrupted and proximal His with hydrophobicity <0; unclear when these parameters are mixed. ^{#}Light grey box: Experimental data on flunarizine sensitivity are consistent with prediction based on analysis of central hydrophobicity and proximal His residue. ^{##}With box: Resolution of data insufficient to judge flunarizine sensitivity due to lack of IC50 value ^{###}Light blue box: Prediction is unclear due to mixed parameters (central hydrophobicity; proximal His) | | | | | | | |

The E1 sequence of the strain J6, AF177036 is shown as Seq. ID. No.1.
Interestingly, in the flunarizine sensitive J6-E1 which has stringent requirements for pH-triggered fusion, two His in proximity of the putative fusion loop are embedded within a large continuous hydrophobic region. In contrast, in strains with natural resistance to flunarizine and with more relaxed requirement for membrane fusion (e.g. J8 [GT2b], S52 [GT3a], and S13 [GT5a] the His residue proximal to the putative fusion loop is in a much more hydrophilic environment and the continuity of the hydrophobic region is disrupted (Fig. 5A-D). Replacement of four E1-R3.2 residues by amino acids conserved among flunarizine resistant GT2b isolates (J6/J8-E1R3.2-4aa) also disrupts continuity of this hydrophobic region and increases hydrophilicity close to these two His residues (Fig. 5G). Notably, the modified E1 protein (J6/J8-E1R3.2-4aa) not only displays resistance to flunarizine, it also has broadened requirements for low pH-triggered membrane fusion (Fig. 4), thus reinforcing this correlation. In contrast, exchange of the four residues within the E1R.1 region (J6/J8-E1R3.1-4aa) slightly reduces hydrophobicity of the putative fusion loop but does not affect resistance to flunarizine nor alter low pH fusion triggering (Fig. 5F). Collectively, these observations support a model where a central hydrophobic region directly downstream of the putative fusion loop and containing at least one His residue controls pH-dependence of HCV membrane fusion and, correlating with this, sensitivity to HCV membrane fusion inhibitors. The level of predicted hydrophobicity of the most proximal downstream His and continuity of the central hydrophobic region predicts flunarizine sensitivity. All HCV strains showing a continuous central hydrophobic region and also a hydrophobicity greater than 0 around the proximal His are sensitive to flunarizine (Table 2). In contrast, strains with a disrupted central hydrophobic region and also hydrophobicity lower than 0 around the proximal His residue are resistant to flunarizine (Table 2). Thus, for all 11 strains with robust experimental data on flunarizine sensitivity, this simple algorithm stratifies strains into sensitive and in-sensitive strains.

### DISCUSSION

Here we demonstrate the mode of action of several chemically related, HCV entry inhibitors, identified novel derivatives with improved efficacy, defined viral determinants of resistance, mapped the viral target site of these molecules and advanced our understanding how HCV escapes this class of drugs. Moreover, this work also sheds light on E1 protein features that control pH dependent membrane fusion.

Several laboratories reported a broad set of loosely related compounds from the families of diphenylpiperazines, diphenylpiperidines, phenothiazines, thioxanthenes, and cycloheptenepiperidines as potent HCV entry inhibitors. Here we unite and extend these reports by showing that representatives of these chemical classes share a common mode of action which is specific inhibition of HCV membrane fusion. This conclusion is based on several observations: First, an HCV variant with resistance mutations to flunarizine exhibited pronounced cross-resistance to all tested representatives of these chemical scaffolds. In contrast these mutations did not confer resistance to a previously published entry inhibitor with grossly different scaffold (i.e. curcumin). Second, all tested representatives of these compound families were uniquely able to fully inhibit HCV infection when administered only during low pH triggering of the HCV fusion process. In contrast, BJ486K, an HCV entry inhibitor with different chemical properties and alternative mode of action only exerted antiviral activity when present throughout the viral entry process. Finally, flunarizine, *p*-methoxy-flunarizine, and chlorcyclizine, the most potent among these related molecules and also the related compounds pimozide, trifluoperazine and fluphenazine share a pronounced preference for inhibition of GT2-derived viral strains. These findings suggest that most GT2 strains are particularly vulnerable to this novel class of HCV membrane fusion inhibitors. The specific sensitivity of GT2 viruses to these compounds seems to be linked with a uniquely tight regulation of pH-dependent membrane fusion and/or specific sequence properties of their E1 proteins.

Based on these observations we completed a structure-activity relationship analysis and identified *p*-methoxy-flunarizine, as most potent compound of this series of inhibitors. p-Methoxy-flunarizine exhibited an IC₅₀ of 28 nM, which is 8-fold better than flunarizine and ca. 2-fold improved compared to chlorcyclizine. Notably, this modification does not alter cross-genotype coverage of the antiviral activity. This is in line with our previous observation that flunarizine-related molecules with modifications in the tricyclic aromatic phenothiazine backbone exhibited somewhat improved cross-genotype antiviral activity (fluphenazine and trifluoperazine. This broad analysis of related compounds permits a more precise definition of the chemical space of these membrane fusion inhibitors. Our analysis particularly demonstrates the importance of the cinnamyl group as attractive element for expanding the SAR-studies for achieving improved antiviral properties. The possibility of synthesizing azido- and 3-(trifluoromethyl)-3*H*-diazirine derivatives (table 1, entries 4 and 14) paves the way for initiating studies on target elucidation for this group of potential antiviral agents.

Identification of viral determinants for susceptibility to these compounds is important to select patients that may benefit from treatments involving such molecules. This information is also critical to guide development of improved membrane fusion inhibitors with regard to potency and possibly also cross-genotype coverage. By using a series of chimeras between the sensitive J6 strain and a resistant GT2b isolate J8 we pinpointed viral determinants of flunarizine sensitivity. We identified a central hydrophobic region in J6-E1 (J6-E1-R3.2; Aa 290-312) to be most important for controlling sensitivity to this compound. In fact, four residues within this region and which are conserved among sensitive GT2a strains (M/V290, W299, V301, P310) and where resistant GT2b strains encode a conserved alternative reside (A290, N299, T301, Q310) cause and 8-fold, 6-fold or 27-fold shift of the IC50 of flunarizine, chlorcyclizine and *p-*methoxy-flunarizine, respectively. Notably, these amino acids also influence responsiveness of J6 E1 to low pH-triggered membrane fusion. While parental J6 is sensitive to fluanrizine and fuses only in presence of very low pH, J6/J8-E1R3.2_4aa is much more resistant and it accepts a more neutral pH for membrane fusion (Fig. 5). These changes are accompanied by a remarkable alteration of hydrophobicity in the central hydrophobic region directly downstream of the putative fusion loop. In the mutant J6 protein (J6/J8-E1R3.2_4aa) the hydrophobic stretch is disrupted and the two proximal His residues end up in a more hydrophilic environment. Notably, HCV strains that have natural resistance to flunarizine also display a disrupted central area of hydrophobicity and their His residue most closely downstream of the putative fusion loop is in a more hydrophilic environment. Taken together these findings suggest that analysis of the hydrophobicity within the E1 region proximal to the putative fusion loop including the assessment of the hydrophobicity around the adjacent His predicts viral sensitivity to flunarizine and related membrane fusion inhibitors. Beyond this, our data demonstrate that viral determinants in this E1 region control requirements for low pH-dependent membrane fusion, possibly by modifying the accessibility and protonation of His residues close to the putative fusion loop. Therefore, these findings provide an algorithm for selection of HCV patients that may benefit from treatments with membrane fusion inhibitors. They also provide new perspectives for development of further improved inhibitors and the offer new insights into the molecular mechanisms that control HCV membrane fusion.

## Claims

1. A compound of the general formula I 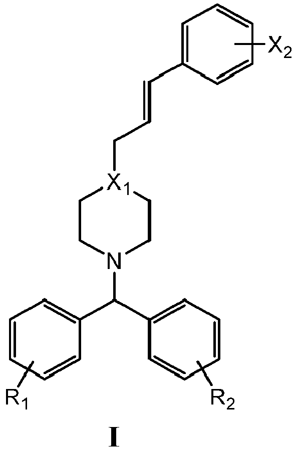
wherein R₁ and R₂ are independently from each other selected from a group of H, Halogen or a C₁ - C₄ alkyl group whereby at least one of R1 and R2 is a halogen, R₁ and/or R₂ may be present once, twice, three, four or five times at the aryl group,
X₁ is C or N;
X₂ is selected from a OR₃ group or a NR₄R₅ group wherein
R₃ is H or a C1 - C4 alkyl group, and X₂ may be present once, twice, three, four or five times at the aryl group;
R₄ and R₅ are independently from each other selected from the group of H, N, C₁ - C₄ alkyl group, and (CH₂)ₙ - CR₆R₇R₈;
wherein R₆ and R₇ are independently from each other selected from H or a C₁ - C₄ alkyl group, and
R₈ is H, C₁ - C₄ alkyl group or a cyclopropanyl group;
n is 0 or 1;
or salts, solvates or hydrates thereof.

2. The compound of general formula I according to claim 1 wherein X₁ is N.

3. The compound of general formula I according to claim 1 or 2 wherein the halogen of R₁ and R₂ is F.

4. The compound of general formula I according to any one of the preceding claims wherein X₂ is a C₁ - C₄ alkoxy group, in particular, a methoxy group.

5. The compound of general formula I according to any one of the preceding claims wherein X₂ is a NR₄R₅ group wherein at least one of R₄ and R₅ is H.

6. The compound of general formula I according to any one of claims 1 to 4 wherein NR₄R₅ is a group selected from NH₂, N₃, NH-CH(CH₂)₂, NH-C-tertbutyl, NH-ethylclyclopropyl.

7. The compound of general formula I according to any one of the preceding claims being a compound of

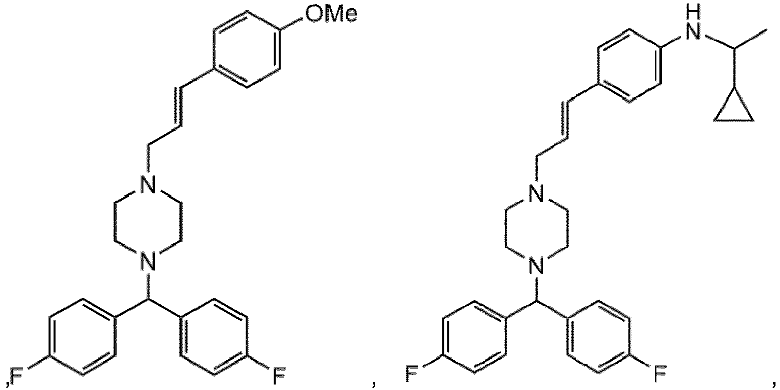

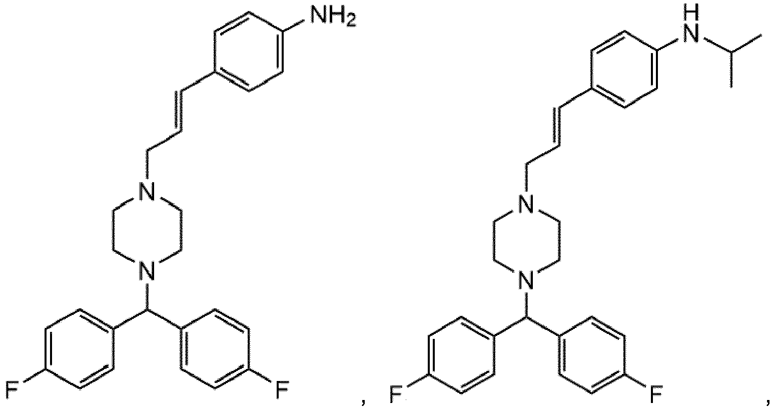

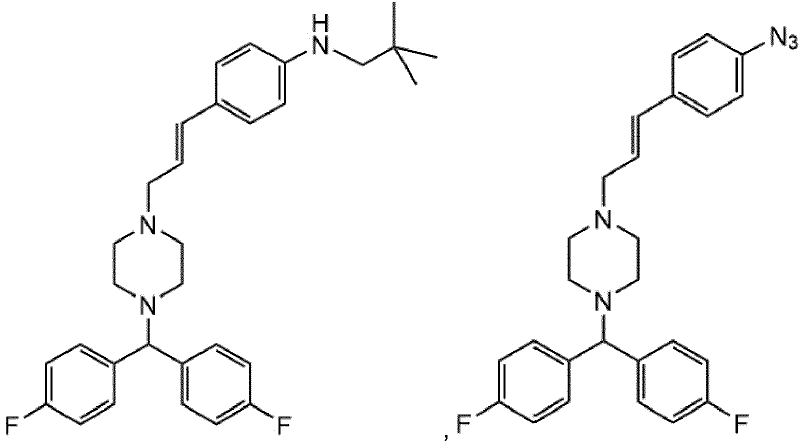

8. A pharmaceutical composition containing a compound of general formula I according to any one of claims 1 to 7.

9. The compound of general formula I according to any one of claims 1 to 7 or the pharmaceutical composition according to claim 8 for use in preventing or treating hepacivirus infection including non-primate hepacivirus like non-primate hepacivirus from horse, rat, gorilla, rodent, bat, bovine or hepatitis C virus.

10. The compound of general formula I or the pharmaceutical composition for use according to claim 9 for use in preventing or treating hepatitis C virus infection, in particular, wherein the HCV is HCV of genotype 2.

11. The compound of general formula I or of the pharmaceutical composition for use according to any one of claims 9 to 10 in combination with a further anti-hepacivirus inhibitor, like an anti-Hepatitis C virus inhibitor.

12. A method for determining effectiveness of prophylactic or therapeutic treatment of hepaci virus infection, like HCV infection, in particular, HCV type 2 infection, comprising:
- Determining the interfacial hydrophobicity of the E1 of the hepaci virus, like E1 of the HCV strain, using the Wimley and White interfacial hydrophobicity scale;
- Determining the sensitivity to a diphenylpiperazine or diphenylpiperidine based hepaci virus inhibitor, as well as to a phenothiazine and cycloheptenepiperidine based hepaci virus inhibitors, in particular, a compound of any one of claims 1 to 7,
wherein a disrupted central hydrophobic region of the E1 protein or a hydrophobicity of the E1 protein lower than zero at the next following His residue as has been determined with the Wimley-White hydropathy plot is indicative for reduced sensitivity to a diphenylpiperazine or diphenylpiperidine based hepaci virus inhibitor as well as phenothiazine and cycloheptenepiperidine based hepaci virus inhibitors.

13. A method for determining the therapy regimen of an individual afflicted with hepaci virus infection or being at risk of being afflicted with hepaci virus infection, in particular, having or being at risk of hepaci virus infection, like of HCV infection, comprising the step of
- Providing a sample obtained from said individual and determining the interfacial hydrophobicity of the E1 protein of the hepaci virus, like the E1 of the HCV or its homolog, in particular, using the Wimley-White hydropathy plot;
- Analyzing whether the central hydrophobicity region is disrupted or the hydrophobicity is below zero at the next following His residue, in particular, the hydrophobicity is below zero applying the Wimley-White hydrophathy plot;
- Determining the therapy regimen wherein no inhibitor of the E1 protein of hepaci virus, like of HCV or its homolog, in particular, a diphenylpiperazine or diphenylpiperidine based hepaci virus inhibitor as well as phenothiazine and cycloheptenepiperidine based hepaci virus inhibitors is applied when the central hydrophobicity region is disturbed or the hydrophobicity is below zero at the next following His residue applying the Wimley-White hydropathy plot.

14. The method according to claim 13 wherein a fusion inhibitor, in particular, a diphenylpiperazine or diphenylpiperidine based hepaci virus inhibitor, like HCV inhibitor as well as phenothiazine and cycloheptenepiperidine based hepaci virus inhibitors like a compound according to general formula I or a pharmaceutical composition containing the same, is applied when the central hydrophobic region of E1 is not disturbed or the hydrophobicity is zero or above zero applying the Wimley-White hydrophathy plot.

15. The method according to any one of claims 12 to 14 wherein the central hydrophobicity region of the E1 protein include the region of aa 290 to aa 312 of SEQ. ID No.1, in particular, determining whether the HCV strain contain mutations at positions 290, 299, 301 and 310 of SEQ. ID No. 1 which disturb the central hydrophobic region of E1 and the presence of said mutations results in a hydrophobicity below zero applying the Wimley-White hydropathy plot.
